# EUROPEAN PATENT APPLICATION

(11) **EP 1 435 263 A1**
(43) Date of publication of application: **07.07.2004**
(21) Application number: 02801509.7
(22) Date of filing: 09.10.2002
(51) Int. Cl.: B01J 35/02

(54) **PHOTOCATALYTIC MATERIAL SELECTIVELY INACTIVATING BIOLOGICALLY HARMFUL SUBSTANCE AND UTILIZATION THEREOF**

(30) Priority: 10.10.2001 JP 2001313174; 18.10.2001 JP 2001321168; 18.10.2001 JP 2001321169; 18.10.2001 JP 2001321170
(71) Applicant: NORITAKE CO., LIMITED, Nagoya-shi, Aichi 451-8501 (JP); Yamaguchi, Koushi, Koganei-shi, Tokyo 184-0004 (JP)
(72) Inventor: YAMAGUCHI, Koushi, Koganei-shi, Tokyo 184-0004 (JP); KONDO, Yoichi, c/o NORITAKE CO., LIMITED, Nagoya-shi, Aichi 451-8501 (JP); KUROBE, Hisanori, c/o NORITAKE CO., LIMITED, Nagoya-shi, Aichi 451-8501 (JP); KATO, Shinji, c/o NORITAKE CO., LIMITED, Nagoya-shi, Aichi 451-8501 (JP); WATANABE, Hirokazu, c/o NORITAKE CO., LIMITED, Nagoya-shi, Aichi 451-8501 (JP); IWATA, Misao, c/o NORITAKE CO., LIMITED, Nagoya-shi, Aichi 451-8501 (JP)
(74) Representative: Bannerman, David Gardner
(86) International application number: PCT/JP2002/010462
(87) International publication number: WO 2003/033143

(57) **Abstract**

The present invention relates to a material that is able to selectively inactivate a specific biologically harmful substance through photocatalysis, and uses thereof.

The photocatalytic material (4) provided by the present invention comprises a holding substance (8) having holding specificity of selectively holding a specific biologically harmful substance (2), a photocatalyst (5) that is able to inactivate the harmful substance (2) held by the holding substance (8) through photocatalysis, and bridging molecules (7) that link the holding substance (8) to the photocatalyst (5), the bridging molecules (7) being arranged on the surface of the photocatalyst (5) in the form of a monolayer.

## Description

### TECHNICAL FIELD

The present invention relates to a material (composition) that selectively inactivates a biologically harmful substance such as a virus, a bacterium, or a toxin through photocatalysis, a method of manufacturing the material, and a harmful substance treatment apparatus constructed using the material.

### BACKGROUND ART

To prevent contamination of biological or medical/pharmacological samples such as blood or blood preparations with organisms such as pathogenic bacteria and viruses that are biologically hazardous, or toxins or the like produced by such organisms (hereinafter these are referred to collectively as 'biologically harmful substances' or merely 'harmful substances'), treatment to inactivate such harmful substances or treatment to separate out and thus eliminate such harmful substances is carried out on these samples.

Of these treatments, treatment to eliminate harmful substances by filtration and treatment to inactivate harmful substances by heating or the like are widely carried out on samples containing biochemical raw material substances such as blood preparations. However, treatment to inactivate by heating, electrolysis or the like is undesirable, since there is a risk of denaturing not only the harmful substances such as viruses and toxins but also principal components of the sample such as proteins. Moreover, with the method of physically separating out and eliminating by filtration, it is difficult to completely eliminate harmful substances of varying sizes (in particular microscopic harmful substances).

In recent years, as a method of inactivating harmful substances instead of conventional heating, electrolysis or the like, a method in which a transition metal oxide (titanium dioxide etc.) or other semiconductor substance that acts as a photocatalyst is used has received attention. For example, Japanese Patent Application Laid-open No. 8-23970 and Japanese Patent Application Laid-open No. 2000-41667 describe methods in which harmful substances such as viruses are inactivated using a photocatalyst such as titanium dioxide.

The harmful substance inactivation method described in Japanese Patent Application Laid-open No. 8-23970 is characterized in that fine particles of the photocatalyst (titanium dioxide etc.) are added to and dispersed in a liquid such as blood, and the dispersion is irradiated with light to inactivate viruses or the like in the liquid. With this method, a step of separating the fine particles of the photocatalyst out from the liquid is required after the irradiation with light, and the harmful substance inactivation treatment is complicated. Moreover, there is a disadvantage that components (proteins etc.) contained in the liquid sample such as blood are denatured or decomposed by the strong oxidizing power of the fine particles of the photocatalyst such as titanium dioxide.

On the other hand, the harmful substance inactivation method described in Japanese Patent Application Laid-open No. 2000-41667 is characterized in that a photocatalytic material such as titanium dioxide is held in advance on the surface of a substrate that is able to come into contact with blood or a blood preparation, and then light is irradiated onto the photocatalyst-containing substrate to inactivate harmful substances such as viruses contaminating the blood or blood preparation. However, even with this method, the disadvantage that components (proteins etc.) contained in the blood or blood preparation contacting the substrate are denatured or decomposed by the strong oxidizing power of the photocatalyst such as titanium dioxide has not been resolved.

### DISCLOSURE OF THE INVENTION

It is an object of the present invention to provide a material (composition) that is able to selectively inactivate one or a plurality of specific biologically harmful substances that may be contained in a liquid or gas (including a vapor or aerosol; likewise hereinafter) that is a sample to be treated, and a method of manufacturing the material. Moreover, it is another object of the present invention to provide a method of selectively and efficiently inactivating one or a plurality of specific harmful substances from a sample to be treated (a liquid or a gas) using such a material. Moreover, it is another object to provide a harmful substance treatment apparatus that can be used for selectively and efficiently inactivating one or a plurality of specific biologically harmful substances contained in a sample to be treated.

A material provided by the present invention used to selectively inactivate one or a plurality of specific biologically harmful substances that may be contained in a liquid or gas to be treated is a photocatalytic material (composition) that exhibits a photocatalytic action. This material (composition) comprises a holding substance having holding (binding) specificity of selectively holding a specific biologically harmful substance, a photocatalyst that is able to inactivate the harmful substance held by the holding substance through photocatalysis, and bridging molecules (this term refers to a moiety that forms a bridge; likewise hereinafter) that link the holding substance to the photocatalyst, the bridging molecules being arranged on the surface of the photocatalyst in the form of a monolayer (i.e. a layer having a thickness corresponding approximately to the size of one molecular structural unit of the pre-designed bridging moiety).

In the present specification, 'photocatalyst' or 'photocatalytic material' refers to a compound for which a photocatalytic reaction occurs upon being irradiated with light. Transition metal oxides such as titanium dioxide and other semiconductors are typical examples included under 'photocatalyst' as defined here.

Moreover, 'specific harmful substance' means, out of biologically harmful substances that contaminate or may contaminate a sample to be treated that takes on the form of either a liquid phase or a gas phase, one or a plurality of harmful substances or parts (fragments) thereof selected as deemed appropriate in accordance with the objective. Moreover, 'inactivate' means to eliminate or markedly reduce the biological hazard of such a harmful substance through the photocatalytic reaction, and includes oxidation, reduction, decomposition and so on of the harmful substance.

The photocatalytic material provided by the present invention having a constitution as described above is a material (composition) that can be suitably used as a harmful substance treating material for inactivating a specific biologically harmful substance from a liquid or gaseous sample to be treated. With the photocatalytic material, a holding substance able to hold (bind) substantially only the specific harmful substance is disposed on the surface of the photocatalyst (typically a transition metal oxide such as titanium dioxide). The specific harmful substance held (captured) by the holding substance can thus be selectively oxidized, reduced or decomposed, and thus inactivated, through the photocatalysis.

Furthermore, with the photocatalytic material of the present invention, the holding substance is linked to the surface of the photocatalyst via bridging molecules that are arranged in the form of a monolayer. The thickness of the layer comprising the bridging molecules (i.e. the monolayer-form bridging moieties) is thus low, and hence the holding substance can be disposed close to the photocatalyst. Consequently, according to the photocatalytic material of the present invention, inactivation of the harmful substance can be carried out efficiently while suppressing effects on (impairment of) the photocatalytic action by the bridging molecules. For example, it is suitable for the thickness of the layer constituted from the bridging molecules formed on the surface of the photocatalyst to be 1 to 2 nm.

Moreover, preferably, the bridging molecules (bridging parts) are bonded to the surface of the photocatalyst by inorganic covalent bonds (i.e. covalent bonds not involving carbon, for example Si-O bonds). Effects of the photocatalytic action on the bridging molecules are reduced by such inorganic bonding. Specifically, detachment of the holding substance and the bridging molecules from the surface of the photocatalyst due to the photocatalytic action can be prevented in advance.

Moreover, in a preferable form of the photocatalytic material of the present invention, a layer comprising the photocatalytic material is formed on a surface of a substrate. There are no particular limitations on the form of the substrate. A substrate having a sheet form, a tubular form, a granular form, or another regular form, or an irregular form can be used in accordance with the usage. A substrate made of a material able to transmit light that can excite a transition metal oxide such as titanium dioxide (typically, ultraviolet radiation of wavelength 250 to 400 nm) is preferable. For example, one photocatalytic material suitable for treating a harmful substance is characterized by having a substrate that will transmit light (typically, ultraviolet radiation), wherein a photocatalyst as described above is formed in the form of a film of thickness approximately 1 to 7 µm on a surface of the substrate. According to a photocatalyst layer (typically, a layer comprising a transition metal oxide such as titanium dioxide) having such a thickness, light of a prescribed wavelength will be absorbed, and hence a sufficient photocatalytic reaction for practical purposes can be brought about. Moreover, there is an advantage that the photocatalyst will not be prone to peeling off from a substrate made of a metal or a ceramic. More preferably, the photocatalyst layer formed in the form of a film has a transmittance to ultraviolet radiation of wavelength 250 to 400 nm of not more than 1%. According to such a low-transmittance photocatalyst layer, strong ultraviolet radiation can be prevented from passing through the photocatalyst layer and being irradiated onto the sample to be treated. Degradation of the quality of the substrate and the sample to be treated by ultraviolet radiation can thus be prevented.

The photocatalytic material (composition) described above can typically be manufactured through a method comprising a step of preparing a photocatalyst that is able to inactivate the harmful substance through photocatalysis, a step of disposing bridging molecules on a surface of the photocatalyst in the form of a monolayer, and a step of bonding a holding substance having holding specificity of selectively holding the specific harmful substance to the bridging molecules.

In this manufacturing method, preferably, in the step of disposing bridging molecules on a surface of the photocatalyst in the form of a monolayer, processing is carried out in which the photocatalyst is exposed to a vapor containing a coupling agent, thus bonding the coupling agent to the surface of the photocatalyst. The compound functioning as the coupling agent typically has a functional group having a substituent that will bond to an organic material, and a hydrolyzable group that will react with an inorganic material. The coupling agent is thus suitable for forming the bridging molecules on the surface of the photocatalyst. For example, it is suitable to use a coupling agent having an amino group, a vinyl group, a methacryl group, a mercapto group or the like as the functional group, and an alkoxy group as the hydrolyzable group. It is particularly suitable to use a silane coupling agent having such a functional group and such an alkoxy group. By exposing the photocatalyst to a vapor containing the coupling agent, molecules of the coupling agent (bridging molecules) can then be arranged densely in the form of a monolayer on the surface of the photocatalyst (typically, a transition metal oxide such as titanium dioxide). For example, as a preferable form, according to the present method, the bridging molecules can be arranged densely side-by-side on the surface of the photocatalyst in a state in which the molecular chains extend in a direction normal (perpendicular) to the surface of the photocatalyst. According to the present method, it is thus possible to manufacture a photocatalytic material characterized in that the holding substance is linked to the surface of the photocatalyst at high density in a state close to the photocatalyst.

Moreover, according to the present invention, there is provided a method of selectively inactivating a specific biologically harmful substance contained in a liquid or gas to be treated. This method is characterized by comprising a step of preparing the photocatalytic material of the present invention, a step of making the liquid or gas to be treated come into contact with at least parts of the photocatalytic material containing the holding substance, and a step of irradiating light capable of bringing about a photocatalytic reaction onto at least parts of the photocatalytic material containing the photocatalyst. According to this method, as a result of using a photocatalytic material of the present invention as described above, it is possible to selectively inactivate a specific harmful substance corresponding to the holding (binding) specificity of the holding substance possessed by the material.

Moreover, according to the present invention, there is provided an apparatus that treats, by photocatalysis, a specific biologically harmful substance contained in a liquid or gas to be treated. This apparatus comprises the photocatalytic material of the present invention, a flow path through which the liquid or gas containing the specific harmful substance is fed onto the photocatalytic material, and a light source that irradiates light capable of bringing about a photocatalytic reaction onto at least parts of the photocatalytic material containing the photocatalyst. According to such an apparatus, by using the photocatalytic material of the present invention in a photocatalytic treatment unit, a prescribed virus, bacterium, toxic substance (typically including a peptide component), autoimmune disease pathogenic factor or the like can be inactivated, and further decomposed or eliminated.

One preferable apparatus provided by the present invention has at least one pair of optically transparent substrates (preferably at least one pair of substrates formed in approximately sheet shapes) disposed separated from one another. A material comprising a holding substance having holding specificity of selectively holding a specific biologically harmful substance, and a photocatalyst that is able to inactivate the harmful substance held by the holding substance through photocatalysis is disposed on both or one of the mutually facing surfaces of the substrates forming each pair. Furthermore, between the substrates forming each pair is disposed a wall member that divides the space formed between the substrates into two. This wall member is provided in a state such that a fluid can flow from one side of the wall member to the other. Furthermore, at least one inflow port through which the liquid or gas to be treated is introduced into the space formed between the substrates forming each pair is formed between one of the substrates and the wall member, and at least one outflow port through which the liquid or gas is discharged from this space to the outside is formed between the other one of the substrates and the wall member. Furthermore, the apparatus has light source that irradiates light capable of bringing about a photocatalytic reaction through one of the substrates onto at least parts of the material containing the photocatalyst. Preferably, the holding substance contained in the material is linked to the surface of the photocatalyst via bridging molecules (more preferably, bridging molecules formed in a monolayer as described above).

With the apparatus having this form, light from the light source is irradiated onto at least parts of the material containing the photocatalyst, thus putting the photocatalyst (typically constituted from a transition metal oxide such as titanium dioxide) into an excited state. In this state, the liquid or gas to be treated is introduced from the inflow port(s) (typically provided at one end between the substrates) into the space between the substrates. As a result, a prescribed harmful substance contained in the sample to be treated can be held (captured) by the holding substance of the photocatalytic material, and can be selectively inactivated by the photocatalytic action of the photocatalytic material. After the treatment by the photocatalyst, the treated sample that was fed from the inflow port(s) into the space between the substrates (i.e. the flow path of the treated sample) is discharged to the outside from the outflow port(s) (typically provided at one end between the substrates (preferably adjacent to the inflow port(s) with the wall member therebetween)). Moreover, by making the space between the substrates forming each pair (the flow path of the treated sample) narrow, a high-efficiency compact photocatalytic treatment unit can be provided. By making the unit compact, the apparatus itself can be made compact.

A particularly preferable apparatus having this form is characterized in that the wall member is formed so as to substantially not transmit light. Through the wall member blocking light, the sample to be treated can be prevented from being exposed excessively to the light from the light source, and hence denaturation of useful components in the sample to be treated by this light can be suppressed.

Moreover, another particularly preferable apparatus having this form is characterized in that a plurality of the inflow ports are provided at one end on one side of the space between the substrates that has been divided into two by the wall member, and a plurality of the outflow ports are provided in one end on the other side of the space between the substrates that has been divided into two. By providing a plurality of each of the inflow ports and the outflow ports in one end in the space between the substrates in this way, the flow of the sample (liquid or gas) to be treated through this space can be regulated, and hence turbulence can be prevented from occurring. The sample to be treated can thus be treated efficiently with no stagnation.

Another preferable apparatus provided by the present invention has a vessel having an optically reflective inner surface. At least one substrate (preferably formed in a tubular shape) that is optically transparent and has formed therein a flow path through which a liquid or gas to be treated can flow is disposed inside the vessel. On the inside of each substrate (i.e. the inside of the flow path) is disposed a material comprising a holding substance having holding specificity of being able to selectively hold a specific biologically harmful substance, and a photocatalyst that is able to inactivate the harmful substance held by the holding substance through photocatalysis. Furthermore, the apparatus has a light source that irradiates light capable of bringing about a photocatalytic reaction through each substrate onto at least parts of the material containing the photocatalyst. Preferably, the holding substance contained in the material is linked to the surface of the photocatalyst via bridging molecules (more preferably, bridging molecules formed in a monolayer as described above). Moreover, the light source is preferably disposed inside the optically reflective vessel.

With the apparatus having this form, light from the light source can be irradiated directly onto at least parts of the material containing the photocatalyst, and moreover light reflected from the inner surface of the vessel can also be irradiated onto the photocatalyst (typically constituted from a transition metal oxide such as titanium dioxide). The light from the light source can thus be used efficiently, and the light can be irradiated approximately uniformly onto the photocatalyst disposed on the substrate(s).

A particularly preferable apparatus having this form is characterized in that the light source is disposed inside the vessel, and a plurality of the substrates are provided close to the light source. By making the distance between the light source and each of the substrates be short, the targeted harmful substance contained in the sample to be treated can be treated (inactivated) efficiently through the photocatalysis. In the case of disposing a plurality of substrates (typically tubular substrates) inside the vessel, it is preferable to dispose the substrates in positions such that the distance from the light source is approximately equal for each of the substrates, so that the photocatalytic treatment ability will be approximately the same for each of the substrates. Alternatively, the plurality of substrates may be connected together in series in a state such that the liquid or gas to be treated can pass therethrough. By connecting the flow paths of the substrates together, the rate of inactivation (or the rate of decomposition or elimination) of the targeted harmful substance contained in the sample to be treated can be improved.

A particularly preferable apparatus provided by the present invention is characterized by having cooling means for suppressing heat discharge from the light source(s). According to this constitution, an inappropriate rise in the temperature of the sample to be treated (e.g. a blood sample, a blood preparation, or a biochemical preparation such as an enzyme solution) due to heat from the light source(s) can be prevented, and hence thermal denaturation of useful components contained in the sample to be treated can be prevented. As the cooling means, a blower (fan) that blows, toward the light source(s), a cooling gas (typically air) that will not impede the irradiation of light onto the substrate(s) (the photocatalytic material) is particularly preferable.

### BRIEF DESCRIPTION OF THE DRAWINGS

FIG 1 is an explanatory drawing showing schematically the microscopic structure of a photocatalytic material according to an embodiment.
FIG 2 is an explanatory drawing showing schematically a mode of use of the photocatalytic material.

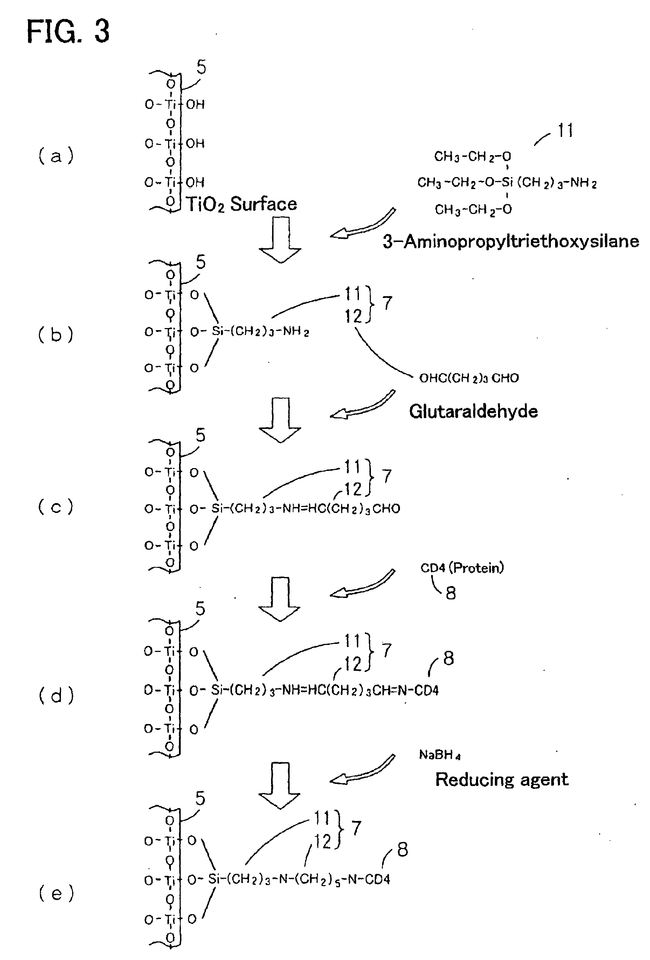

FIGS. 3 are drawings showing schematically a process of the photocatalytic material being manufactured. Specifically, FIG 3(a) is an explanatory drawing showing schematically the state of the surface of a transition metal oxide (titanium dioxide) having a sheet-like form, this being a photocatalyst. FIG. 3(b) is an explanatory drawing showing a state after a silane coupling agent has been introduced onto the transition metal oxide. FIG 3(c) is an explanatory drawing showing a state after glutaraldehyde has been introduced onto the silane coupling agent. FIG 3(d) is an explanatory drawing showing a state after a holding substance (CD4) has been linked to the end of the aldehyde. FIG 3(e) is an explanatory drawing showing a state after the bridging molecules introduced onto the surface of the transition metal oxide have been reduced.
FIG 4 is a graph showing the relationship between the film thickness (µm) of the photocatalyst film in a photocatalytic material and the film formation time (min).
FIG 5 is a graph showing the relationship between the film thickness (µm) of the photocatalyst film in a photocatalytic material and the UV absorptance (%).
FIG 6 is an explanatory drawing showing schematically a mode of use of a photocatalytic material not having a holding substance.
FIG 7 is a graph showing the relationship between the film thickness (µm) of the photocatalyst film in a photocatalytic material and the bacteria reduction rate (%).
FIG 8 is a bar chart showing the relationship between the film thickness (µm) of the photocatalyst film in a photocatalytic material and the amount of a harmful substance (HIV) remaining.
FIG. 9 is an explanatory drawing showing schematically a mode of use of a photocatalytic material according to an embodiment.
FIG. 10 is a bar chart showing the relationship between the film thickness (µm) of the photocatalyst film in a photocatalytic material and the amount of a harmful substance (HIV) remaining.
FIG. 11 is a graph showing the relationship between the film thickness (µm) of the photocatalyst film in a photocatalytic material and the amount of albumin remaining (%).
FIG. 12 is a schematic drawing for explaining a cut tape method used for measuring the strength of attachment of a film of a photocatalyst (transition metal oxide) in a photocatalytic material.
FIG. 13 is a graph showing the relationship between the UV irradiation time (min) and the amount of HIV (HIV inactivation efficiency: %) for the cases of using photocatalytic materials of various forms.
FIG. 14 is a side view showing the constitution of a harmful substance treatment apparatus according to an embodiment.
FIG 15 is a side view showing, from one direction, the constitution of a photocatalytic treatment unit installed in the treatment apparatus shown in FIG. 14.
FIG. 16 is a side view showing, from another direction, the constitution of the photocatalytic treatment unit shown in FIG. 15.
FIG 17 is a side view showing, from one direction, the constitution of a UV lamp unit installed in the treatment apparatus shown in FIG 14.
FIG 18 is a block diagram showing schematically a system for treating a liquid (e.g. blood) to be treated using a harmful substance treatment apparatus according to an embodiment.
FIG. 19 consists of a side view and a plan view showing the constitution of a treatment apparatus according to an embodiment.
FIG. 20 is a perspective view showing, from one direction, the constitution of a photocatalytic treatment unit installed in the treatment apparatus shown in FIG. 19.

### BEST MODE FOR CARRYING OUT THE INVENTION

Preferable embodiments of the present invention will now be described with reference to the drawings. Note that technical matters that are required for carrying out the present invention but are not particularly mentioned in the present specification are matters of design variation that could be apprehended by a person skilled in the art based on prior art. The present invention can be carried out based on the technical details disclosed in the present specification and/or drawings, by referring to common general technical knowledge in the field in question as appropriate.

A photocatalytic material (composition) of the present invention is a material that has as principal constituent elements thereof a holding substance having holding specificity of selectively holding a specific biologically harmful substance, and a photocatalyst that is able to inactivate the harmful substance held by the holding substance through photocatalysis. Of these, the photocatalyst should be a compound that can bring about a photocatalytic reaction upon absorbing ultraviolet radiation or the like. For example, a transition metal oxide or another semiconductor substance is suitable. Titanium dioxide is particularly preferable.

The form of the photocatalyst can be changed as appropriate in accordance with the usage and the mode of use, with there being no limitations so long as the form is such that the photocatalyst can contact the sample to be treated efficiently. For example, in the case that the sample to be treated is a liquid, a sheet form, a film form, a tubular form, beads (a spherical form), a honeycomb form, or a porous form like a sponge is suitable. In the case that the sample to be treated is a gas, a tubular form, a honeycomb form, or a porous form like a sponge is suitable. Typically, the photocatalytic material of the present invention includes a metallic or ceramic substrate (support), and the photocatalyst is formed as a layer (film) on the surface of the substrate. There are no particular limitations on the form of the substrate, with it being possible to use a sheet form, a tubular form, beads (a spherical form), a honeycomb form, or a porous form like a sponge in accordance with the usage. A substrate having good optical transparency (e.g. a glass substrate) is preferable.

A conventional publicly known film formation method can be used with no particular limitations to form the film-like photocatalyst layer on the surface of the substrate (including the internal walls of the pores in the case of a porous form). For example, a photocatalyst layer (thin film) made of titanium dioxide or the like can be formed on the surface of a ceramic or metallic substrate using a sputtering method, an ion plating method, an electron beam deposition method, a chemical vapor deposition (CVD) method, a spray coating method, a dip coating method, a sol-gel method or the like. A preferable film formation method is CVD, with normal pressure (atmospheric pressure) CVD in which a vapor phase chemical reaction is carried out under atmospheric pressure being particularly preferable. For example, a mist containing a raw material compound (typically an organometallic compound such as a titanium alkoxide) that has been made into fine droplets through ultrasonic treatment is subjected to thermal decomposition at high temperature and vapor transport, and the decomposition product (typically a metal oxide) is accumulated on the substrate, which has been heated to approximately 400°C to 550°C or above. As a result, a photocatalyst layer (film) made of a metal oxide such as titanium dioxide can be formed fairly evenly over a prescribed region of the substrate surface.

Examples of the holding substance in the photocatalytic material of the present invention include various antibody molecules and antibody fragments, and receptors possessed by tissue or cells that act as the host of a prescribed virus or bacterium (i.e. substances to which a virus or bacterial toxin that is a harmful substance binds specifically) and receptor fragments.

For example, antibodies for antigenic substances present at a prescribed site of a bacterium (outer membrane, capsule, flagellum, etc.) as shown in Table 1 can be suitably used as the holding substance.

**Table 1**

| | | |
|---|---|---|
| Antigen | Site | Antibody |
| O-antigen | Outer membrane | O-antibody |
| K-antigen | Capsule | K-antibody |
| H-antigen | Flagellum | K-antibody |

**Table 2**

| | | |
|---|---|---|
| Virus | Receptor | Disease |
| Herpesviridae Herpes simplex | Nerve cell surface antigen | Encephalitis |
| Hepadnaviridae Hepatitis B virus | Liver cell surface antigen | Hepatitis, liver cancer |
| Picornaviridae Poliovirus | Nerve cell surface antigen | Encephalitis, myelitis |
| Togaviridae Alphavirus | Nerve cell surface antigen | Encephalitis |
| Flaviviridae Yellow fever virus Hepatitis C virus | Liver cell surface antigen Liver cell surface antigen | Acute liver failure (necrosis),hemorrhage Hepatitis, liver cancer |
| Rhabdoviridae Rabies virus | Nerve cell surface antigen | Encephalitis, myelitis |
| Filoviridae Marburg virus Ebola virus | Liver cell surface antigen Liver cell surface antigen | Acute liver failure (necrosis),hemorrhage Acute liver failure (necrosis),hemorrhage |
| Arenaviridae Lassa virus | Lung, liver, nerve cell surface antigen | Interstitial pneumonia, hepatitis, encephalitis, hemorrhage |
| Bunyaviridae Crimean-Congo hemorrhagic fever Hemorrhagic fever with renal syndrome | Lung, liver, kidney cell surface antigen Lung, liver, kidney cell surface antigen | Pneumonia, hepatitis, nephritis, hemorrhage Pneumonia, hepatitis, nephritis, hemorrhage |
| Retroviridae Human immunodeficiency virus (HIV) | T-cell surface CD4 antigen | Acquired immunodeficiency |

**Table 3**

| | | | |
|---|---|---|---|
| Name of toxin | Producing bacteria | Disease | Antibody |
| Endotoxin | Common to Gram-negative bacteria | Endotoxin shock, Disseminated intravascular coagulation | Anti-endotoxin antibody |
| Verotoxin | E. coli O-157 | Intestinal hemorrhage, Hemolytic uremic syndrome | Anti-verotoxin antibody |
| Alpha toxin | Staphylococcus aureus | Dermonecrosis, hemolysis | Anti-alpha toxin antibody |
| Leukocidin | Staphylococcus aureus | Leukocyte destruction | Anti-leukocidin antibody |
| Enterotoxin (SEA, SEB) | Staphylococcus aureus | Food poisoning Contributes to atopic dermatitis | Anti-SEA antibody Anti-SEB antibody |
| Exfoliative toxin | Staphylococcus aureus | Scalded skin syndrome | Anti-exfoliative toxin antibody |
| Toxic shock syndrome toxin (TSST) | Staphylococcus aureus | Shock Shock | Anti-TSST antibody antibody |
| Streptococcal toxic shock syndrome toxin (STSS) | Group A Streptococcus | Shock | Anti-STTS antibody |
| Botulinus toxin | Clostridium botulinum | Flaccid paralysis | Anti-Clostridium botulinum (A-G) antibody |
| Tetanospamin | Clostridium tetani | Spastic paralysis | Anti-tetanospamin antibody |
| Diphtheria toxin | Corynebacterium diphtheriae | Heart failure, peripheral vascular motor nerve paralysis | Anti-diphtheria toxin (A,B) antibody |

Alternatively, receptors that exhibit strong binding ability to part of a pathogenic virus as shown in Table 2 or anti-virus antibodies or analog substances that can be regarded as immunologically equivalent to such receptors can be suitably used as the holding substance. Alternatively, antibodies against bacterial toxins as shown in Table 3 can be suitably used as the holding substance. Note that regarding the holding of the harmful substance by the holding substance, no distinction is made between physical binding such as adsorption and chemical binding such as covalent bonding; the holding may be of any form (binding form) that fastens the harmful substance to the holding substance.

By using any of the antibodies or receptors shown in the above tables (or a man-made analog substance) as the holding substance, a harmful substance that binds specifically to the holding substance used (examples are given in the tables) can be targeted for treatment by the photocatalytic material of the present invention (i.e. can be made to be a target substance to be inactivated). As toxins, in addition to the various bacterial toxins shown in Table 3, any toxin that exhibits a specific antigenicity can be targeted, for example globefish poison (tetrodotoxin), snake venoms, scorpion and spider venoms, and insect venoms such as bee venom.

For example, with bacterial cells, there are three main types of site that exhibit strong antigenicity as shown in Table 1, and furthermore the antigenicity varies according to the type of bacteria. A specific type of bacteria can thus be treated (inactivated) selectively in accordance with the details of the holding substance. For example, by using an antibody that binds specifically to the O-antigen possessed by the O-157 strain of E. coli as the holding substance, this strain can be selectively held and subjected to the photocatalytic treatment.

Alternatively, by using an antibody or the like against an antigenic site possessed in common by a broad range of bacteria or viruses as the holding substance, rather than limiting to a specific type, a relatively broad range of types of bacteria (e.g. all bacteria that are negative under Gram staining) or viruses (e.g. viruses that belong to the Flaviviridae family) can be made to be the specific biologically harmful substances that are targeted.

Moreover, the holding substance is not limited to being one type, but rather two or more holding substances may be used. For example, by using an antibody that binds specifically to the outer membrane or flagellum of a certain type of bacterium and an antibody that binds specifically to a toxin (protein) that is produced by this bacterium and secreted outside the bacterial cells together as holding substances, the bacterium and the toxin can both be selectively treated by photocatalysis as specific harmful substances from a prescribed sample to be treated (a blood sample, liquid food, etc.).

With the photocatalytic material of the present invention, typically, a holding substance as described above is linked to the surface of the photocatalyst via bridging molecules. Bridging molecules suitable for this purpose can be constituted from a compound (typically straight chain molecules) having a hydrolyzable group (a halogen group, an alkoxy group, etc.) able to bond to the inorganic compound (transition metal oxide) that is the photocatalyst, and a functional group (an amino group, a vinyl group, an epoxy group, a methacryl group, a mercapto group, etc.) able to bond to the organic compound that is the holding substance. In general, a compound that is used as a coupling agent is suitable. A silane coupling agent having an amino group, for example an aminoalkylethoxysilane such as 3-aminopropyltriethoxysilane, or an aminoalkylmethoxysilane such as 3-aminopropyltrimethoxysilane, p-aminophenyltrimethoxysilane or N-2-aminoethyl-3-aminopropyltrimethoxysilane, can be preferably used to constitute the bridging molecules (bridging moieties).

Any of various conventional publicly known methods can be used to bond the coupling agent to the surface of the transition metal oxide (photocatalyst) such as titanium dioxide. Preferably, the transition metal oxide such as titanium dioxide that functions as the photocatalyst is exposed to a vapor phase containing a suitable coupling agent. Typically, a hermetically sealed vessel (preferably a gas-tight vessel that can be subjected to pressure reduction) containing dried air or an inert gas is prepared. Next, a low-vapor-pressure solvent (preferably an organic solvent substantially not containing water, for example dehydrated toluene, an absolute alcohol, etc.) containing a suitable coupling agent (preferably a silane coupling agent) as a solute, and the photocatalyst (typically a transition metal oxide such as titanium dioxide) in a prescribed form or a substrate having a film-like photocatalyst layer on the surface thereof are put into the vessel. The inside of the vessel is then preferably subjected to heating and/or pressure reduction, thus generating a vapor of the solvent containing the coupling agent inside the vessel. As a result, a vapor phase of the solvent containing the coupling agent is formed inside the vessel, and hence the photocatalyst in the prescribed form or the substrate having the photocatalyst layer housed inside the vessel is exposed to the vapor. By carrying out this vapor exposure treatment, the coupling agent in the vapor can be bonded in the form of a monolayer onto the surface of the photocatalyst (transition metal oxide). The time required for the vapor exposure treatment and the temperature setting for bringing about a suitable coupling reaction can be changed as appropriate in accordance with the composition and concentration of the coupling agent contained in the vapor, and should be adjusted as appropriate in accordance with the composition and surface form of the photocatalyst used. For example, by carrying out surface analysis on the photocatalyst (transition metal oxide) using an atomic force microscope (AFM) or a scanning tunneling microscope (STM), or by measuring the thickness of the coupling agent layer formed on the surface of the photocatalyst using ellipsometry, it can be determined whether or not the coupling agent layer formed on the surface of the photocatalyst is in the form of a monolayer. The conditions under which the vapor exposure treatment is carried out (the concentration of the coupling agent in the vapor, the temperature inside the vessel, the treatment time, etc.) can then easily be optimized based on the results of the surface analysis using an AFM or an STM and/or the results of the measurement using a spectral ellipsometry system.

From the viewpoint of being able to bond the silane coupling agent or the like to the surface of the photocatalyst at high density, it is preferable to use a transition metal oxide that has hydroxyl groups on the surface thereof in air at normal temperature (e.g. titanium dioxide), but there is no limitation to a transition metal oxide having such a property. When carrying out the present invention, in the case of using a semiconductor substance not having many hydroxyl groups present on the surface thereof as the photocatalyst, before carrying out the vapor exposure treatment described above, it is preferable to treat the surface of the photocatalyst with a suitable acid, thus forming a large number of hydroxyl groups on the surface thereof.

In the case of using a protein-based holding substance such as an antibody or a fragment thereof or a holding substance that is non-protein but has free amino groups, it is convenient to bond amino groups possessed by the holding substance to the bridging molecules. It is thus preferable to introduce a functional group (e.g. an aldehyde group or a carboxyl group) that will readily bond to an amino group onto one end of each of the bridging molecules in advance. For example, by bonding an aldehyde compound such as glutaraldehyde to the terminal amino group of one of the silane coupling agents listed earlier (3-aminopropyltriethoxysilane etc.), an aldehyde group (which will readily bond to an amino group of the holding substance) can be introduced onto the end of the bonding molecular chain, i.e. the end of each of the bridging molecules. As the method of bonding the aldehyde compound to the terminal amino group of the silane coupling agent or the like, any of various conventional publicly known methods may be used. For example, by immersing the photocatalyst or substrate onto the surface of which the silane coupling agent has been introduced in an aqueous solution containing glutaraldehyde, the glutaraldehyde can be bonded to the terminal amino group of the silane coupling agent.

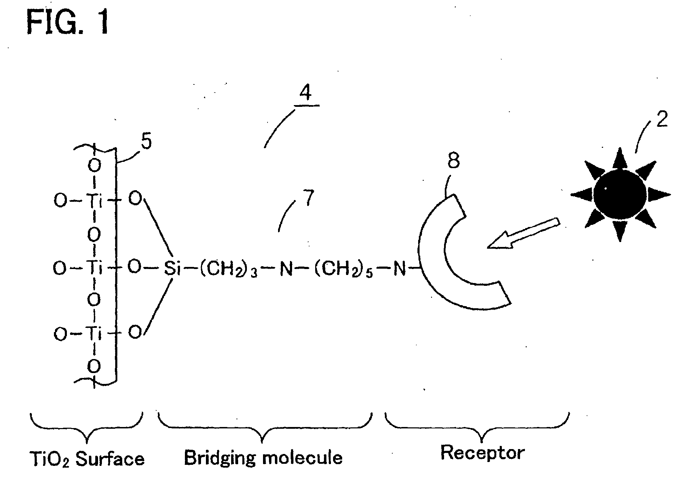

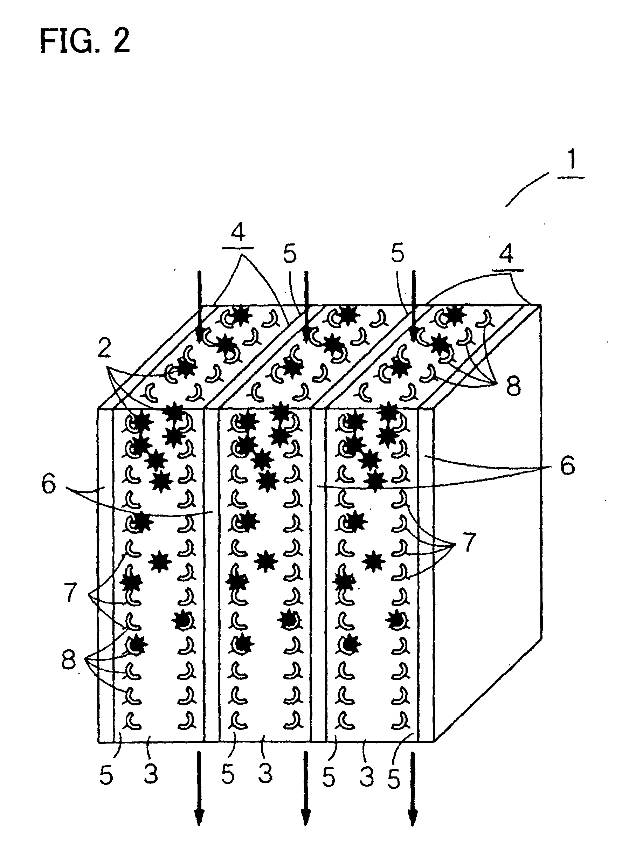

By using materials and carrying out processes as described above, typically a photocatalytic material 4 as shown schematically in FIG 1 (i.e. a material 4 that is able to selectively inactivate a specific harmful substance 2 through photocatalysis) can be obtained. The photocatalytic material 4 shown in FIG 1 is constituted from a film of a photocatalyst 5 (here, titanium dioxide, which is a transition metal oxide) formed on a surface of a sheet-shaped substrate, not shown in FIG 1 (see FIG 2), bridging molecules 7 that are derived from a silane coupling agent and glutaraldehyde and are bonded to the surface of the photocatalyst 5, and a holding substance 8 (i.e. a receptor that is able to bind selectively to a specific harmful substance 2) that is bonded to the end of the molecular chain of each of the bridging molecules 7. The bridging molecules 7 are arranged on the surface of the photocatalyst 5 in the form of a monolayer (a monomolecular film). In this case, the thickness of the layer of the bridging molecules 7 will be approximately equal to the length of each of the bridging molecules 7.

The present invention will now be described in more detail through examples.

FIG 2 is a drawing showing schematically a preferable mode of use of the photocatalytic material 4 shown in FIG. 1. As shown in FIG 2, a plurality of sheets of the photocatalytic material 4 having a shape corresponding to the shape of substrates 6 are prepared, and these photocatalytic materials 4 are disposed parallel to one another with spaces provided therebetween such that the photocatalyst layers (films) 5 face one another. As a result of this constitution, with this treatment apparatus 1, flow paths (treatment chambers) 3 through which a liquid or gaseous sample to be treated containing the targeted harmful substance 2 is fed in from the outside are formed between the photocatalytic materials. Photocatalyst layers 5 face onto each of the flow paths (treatment chambers) 3 from each side thereof. The sample to be treated flowing through each of the flow paths (treatment chambers) 3 is thus able to come into direct contact with the holding substance 8 bonded to the surface of each photocatalyst layer 5 via the bridging molecules 7.

The sheet-shaped substrates 6 of the photocatalytic material 4 sheets are made of glass made of silicon dioxide (SiO₂), and hence light including ultraviolet radiation can pass therethrough. The treatment apparatus 1 has a light source. This light source is provided in a position such that light can pass through the substrates 6 and be irradiated onto the photocatalyst layers 5 facing the flow paths (treatment chambers) 3. There are no particular limitations on the light source used so long as the light source is one that can irradiate light able to bring about a photocatalytic reaction with the photocatalyst 5. For example, in the case of using a transition metal oxide such as titanium dioxide that can be excited by ultraviolet radiation as the photocatalyst, any of various UV lamps that emit ultraviolet radiation can be used. Moreover, a fluorescent lamp with a peak wavelength in the visible region at approximately 600 nm, a black light having a peak at a wavelength in a range of 300 nm to 420 nm, a low-pressure mercury lamp (also capable of producing ozone) having a peak at approximately 185 nm, or the like can also be suitably used. It is preferable to use a light source having a peak wavelength in a range of approximately 150 nm to approximately 600 nm.

Next, a preferable example of the manufacture of the photocatalytic material 4 in the apparatus 1 shown in FIG 2 will be described with reference to FIG. 3.

First, photocatalyst layers (titanium dioxide films) 5 are formed on the surfaces of silica glass substrates 6 (made by Nippon Silica Glass Co., Ltd.) using normal pressure CVD. Specifically, as shown in FIG. 3(a), by using the normal pressure CVD a titanium dioxide film 5 is first coated to a thickness of approximately 1 to 7 µm onto one surface only of each of the substrates 6, which have been formed in a prescribed shape (10 mm long x 10 mm wide x 0.5 mm thick) from silica glass. The thickness of the titanium dioxide film 5 formed on the surface of each substrate is preferably set so as to satisfy the following two conditions: (1) sufficient ultraviolet radiation can be absorbed by the film 5 to produce a photocatalytic action without useful components (a plasma component, etc.) contained in the sample to be treated being denatured, and hence sufficient antibacterial ability can be maintained; and (2) the film 5 is of a thickness such as not to readily peel off from the substrate 6. A titanium dioxide film 5 of thickness approximately 1 to 7 µm formed through a normal pressure CVD or the like will satisfy these conditions (1) and (2). Moreover, with a titanium dioxide film 5 of such a thickness obtained using such a method, the transmittance of ultraviolet radiation of wavelength 250 to 400 nm irradiated from the rear of the substrate 6 to the surface of the titanium dioxide film 5 will be 1% or less.

Specifically, a titanium alkoxide such as titanium isopropoxide was put into a heating vessel, not shown. A substrate was disposed in advance on a heating stage inside the heating vessel. Nitrogen gas was supplied into the vessel as a carrier gas. The heating vessel was then heated to a suitable degree (typically 77 to 130°C), thus vaporizing the titanium alkoxide, and the vaporized raw material gas was led, along with the carrier gas, over the substrate 6, which had been heated in advance to a suitable degree (typically 350 to 500°C). Through this treatment, titanium oxide obtained through a vapor phase chemical reaction was accumulated on a surface of the substrate 6, whereby a titanium dioxide film 5 of a desired thickness was formed on one surface of the sheet-shaped substrate 6.

At this time, the crystal orientation of the titanium dioxide film 5 can be varied by changing the vaporization temperature of the titanium alkoxide, the heating temperature of the substrate 6, and the flow rate of the carrier gas as appropriate. Moreover, it would be easily appreciated by a person skilled in the art that the thickness of the titanium dioxide film 5 can be controlled by suitably controlling the time of deposition of the titanium dioxide film 5 on the surface of the substrate 6, i.e. the coating time (the time for which the CVD is carried out).

After the formation of the titanium dioxide film (photocatalyst layer) 5, washing was carried out with a mineral acid such as nitric acid, and then drying was carried out, thus introducing hydroxyl groups onto the titanium dioxide molecules positioned on the surface of the titanium dioxide film 5 (see FIG 3(a)).

Next, bridging molecules 7 were introduced onto the surface of the titanium dioxide film (photocatalyst layer) 5. That is, as shown in FIG 3(b), the substrate 6 on which the titanium dioxide film 5 had been formed, and a dehydrated toluene solvent containing 3-aminopropyltriethoxysilane (see reference numeral 11 in FIG. 3), which will constitute part of each of the bridging molecules 7, were put into a hermetically sealed vessel that could be subjected to pressure reduction containing dried air or an inert gas. The inside of the vessel was then subjected to heating and/or pressure reduction, thus generating a vapor of the toluene solvent. As a result, a silane coupling reaction occurs in the vapor phase inside the vessel, and hence as shown in the drawing, the 3-aminopropyltriethoxysilane is chemically bonded to and thus fixed on the surface of the titanium dioxide film 5.

The substrate 6 having the 3-aminopropyltriethoxysilane fixed on the surface thereof was then washed a plurality of times using absolute ethanol that had been obtained through dehydration treatment, anhydrous toluene that had been obtained through dehydration treatment, a 1 mM NaOH aqueous solution, and a 1 mM HNO₃ aqueous solution in this order, and was then finally washed using ultrapure water. After that, the substrate 6 was dried using nitrogen gas.

Here, the film thickness of the 3-aminopropyltriethoxysilane fixed on the surface of the titanium dioxide film 5 was measured using spectral ellipsometry. The result was that the measured value of this film thickness was 1.1 ± 0.1 nm. This measured value agrees well with the length of a 3-aminopropyltriethoxysilane molecule, which is approximately 1.1 nm. It was thus verified that the 3-aminopropyltriethoxysilane was bonded to the surface of the titanium dioxide film 5 in the form of a monolayer (i.e. monolayer adsorption occurred) through the silane coupling treatment described above. Moreover, although inconclusive, it is conjectured that the 3-aminopropyltriethoxysilane molecules were arranged on the surface of the titanium dioxide film 5 regularly, pointing in a direction normal to the surface of the titanium dioxide film 5.

Next, the substrate 6 onto which the 3-aminopropyltriethoxysilane had been introduced was put into a glutaraldehyde solution that had been prepared by adding glutaraldehyde (see reference numeral 12 in FIG 3) to a concentration of 3.0 wt% to a 0.1 M potassium phosphate buffer, and stirring was carried out thoroughly (e.g. 1 to 24 hours) at room temperature. As a result, as shown in FIG 3(c), bridging molecules 7 in which one of the aldehyde groups of the glutaraldehyde is bonded to the terminal amino group of the 3-aminopropyltriethoxysilane were formed.

Next, washing with stirring was carried out thoroughly using a 0.1 M potassium phosphate buffer not containing glutaraldehyde (pH 7.5), then a holding substance 8 that is a protein - here, a T-cell surface protein component CD4 fragment ([Cys(Bzl)]⁸⁴ fragment 81-92, made by Sigma-Aldrich), which is a receptor to which HIV (human immunodeficiency virus) can bind specifically, was used - was added to the phosphate buffer, and then stirring was carried out for 24 hours at 4°C. As a result, as shown in FIG 3(d), the amino group of the CD4 fragment (see reference numeral 8 in FIG. 3) bonds to the aldehyde group at the end of each of the bridging molecules 7.

After the CD4 fragment (hereinafter referred to merely as 'CD4') had been linked to the bridging molecules 7, the substrate 6 was recovered by filtration, and was then washed with an NaCl aqueous solution, and then dehydrated (dried). The substrate 6 was put into a 1M tris-HCl buffer (pH 7.5), and was left for 1 hour at room temperature, thus inactivating the aldehyde groups of the residual glutaraldehyde.

After that, as shown in FIG. 3(e), the bridging molecules are typically subjected to reduction treatment. In the present manufacturing example, NaBH₄ was added to the tris-HCl buffer in which the substrate 6 was immersed, thus reducing the Schiff bases. Specifically, the double bond between the aminoalkylethoxysilane and the glutaraldehyde, and the double bond between the glutaraldehyde and the CD4 were reduced. As a result, the stability of the bridging molecules (bridging parts) 7 can be improved.

In this way, a photocatalytic material 4 having a constitution as shown schematically in FIG 1 was prepared. This photocatalytic material 4 can be suitably used as a medical bioreactor that is able to selectively inactivate a specific harmful substance (here, HIV, which binds specifically to the CD4) from within a biochemical or biological sample.

Next, a preferable method of using the apparatus 1 shown in FIG 2 will be described. A liquid sample that may be contaminated with a harmful substance such as blood or a liquid preparation obtained by fractionating blood components, or a gaseous sample that may be contaminated with a harmful substance such as air is introduced into the flow paths 3. At the same time, ultraviolet radiation capable of exciting the titanium dioxide is irradiated onto the apparatus 1 from the light source, not shown. As described earlier, the substrates 6 are optically transparent, and hence the irradiated ultraviolet radiation passes through the substrates 6 and reaches the photocatalyst layers 5, where the ultraviolet radiation is absorbed by the titanium dioxide. At this time, the targeted harmful substance 2 (here, HIV) contained in the sample flowing through the flow paths 3 can be selectively held by the holding substance 8. As a result, the targeted harmful substance 2 can be selectively held in place close to one of the photocatalyst layers 5. The held harmful substance 2 is then rapidly inactivated by photocatalysis, whereby the harmful substance 2 can be removed efficiently from the sample. Specifically, hydroxy radicals (·OH) are produced from moisture (H₂O) that has come into contact with the surface of each of the titanium dioxide films 5 that has been excited by the ultraviolet radiation, and hence strong oxidation reactions can occur in the vicinity of the titanium dioxide films 5. Moreover, super oxide anions (·O₂⁻) are produced from oxygen that has come into contact with the surface of each of the titanium dioxide films 5 that has been excited by the ultraviolet radiation, and hence strong reduction reactions can occur in the vicinity of the titanium dioxide films 5. The targeted harmful substance can be decomposed through such oxidation reaction(s) and/or reduction reaction(s) (i.e. the photocatalysis).

By using the present treatment apparatus 1, a liquid or gas to be treated can thus be purified, and harm (e.g. outbreak of disease) due to the harmful substance 2 (here, HIV) being present in the sample in an active state can be prevented from occurring.

A number of performance evaluation tests were carried out on photocatalytic materials 4 manufactured as described above.

### (Test Example 1)

A photocatalytic material 4 as described above (FIG 1) was placed in each well of a commercially sold 24-well plate, not shown, such that the titanium dioxide film 5 was on the upper surface, and HIV inactivation treatment was carried out.

HIV (HIV-1) was added to a culture solution (RPMI-1640 medium, made by Sigma Chemical Co.), thus preparing an HIV solution having a p24 antigen concentration of 100 ng/ml. 500 µl of this solution was injected into each well of the 24-well plate.

After that, the 24-well plate was placed in a shaking incubator, not shown, and ultraviolet radiation of wavelength 300 to 400 nm was irradiated onto the 24-well plate for 15, 30, 45 or 60 minutes while shaking. A 'BLB 10' black light made by Toshiba Lighting and Technology Corporation was used as the light source. The intensity of the irradiated ultraviolet radiation at the titanium dioxide film 5 on the surface of each substrate 6 at this time was made to be 350 ± 20 µW/cm² (using a 'UM-360' ultraviolet radiation measuring instrument made by Minolta).

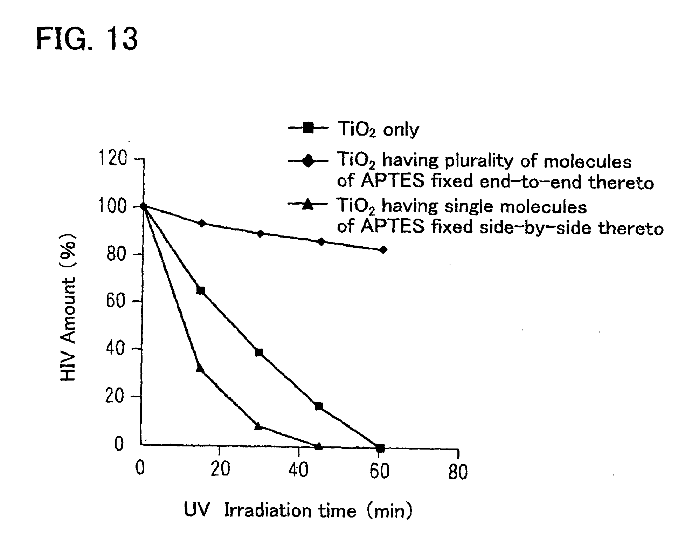

After the ultraviolet radiation had been irradiated for the prescribed time, the HIV solution was recovered from each of the wells of the 24-well plate. The recovered HIV solution was mixed into a culture suspension of H9 cells as host cells, and the mixture of these cells and the HIV solution was cultured for 14 days in an incubator under conditions of 37°C and 5% CO₂. Next, the amount of p24 antigen in the culture solution (supernatant) was measured, thus indirectly measuring the amount of infectious virus remaining after the UV irradiation treatment described above. The results are shown in Table 4 and FIG. 13.

For comparison, in addition to the photocatalytic material manufactured through the process described earlier (FIG 1), a material having the same form but having only the photocatalyst layer formed on the substrate (i.e. a photocatalytic material not having the bridging molecules and the holding substance introduced thereon; listed as 'TiO₂ only' in Table 4) was prepared, and HIV inactivation treatment was carried out as before.

Furthermore, the photocatalytic material manufactured through the process described earlier is a photocatalytic material characterized in that the bridging molecules have been introduced onto the surface of the photocatalyst layer in the form of a monolayer (hereinafter this photocatalytic material is referred to as the 'monolayer-bridged photocatalytic material'), but in contrast to this, a photocatalytic material in which bridging molecules were piled up end-to-end to form a layer thicker than a monolayer on the surface of the photocatalyst layer (i.e. a material in which multilayer adsorption of the coupling agent occurred on the surface of the photocatalyst layer), specifically a photocatalytic material characterized in that the 3-aminopropyltriethoxysilane (APTES) coupling agent has been arranged with two or more molecules thereof linked together on the surface of the photocatalyst layer (hereinafter this photocatalytic material is referred to as the 'multilayer-bridged photocatalytic material'), was prepared, and HIV inactivation treatment was carried out as before. The multilayer-bridged photocatalytic material was prepared as follows.

Carrying out a similar process to in the case of manufacturing the monolayer-bridged photocatalytic material, a photocatalyst layer (titanium dioxide film) of thickness 1 to 7 µm was formed on a surface of a substrate. Next, bridging molecules were introduced onto the surface of the titanium dioxide film. Specifically, the substrate was put into toluene containing 3-aminopropyltriethoxysilane and mixing was carried out, and then refluxing was carried out for a prescribed time. Through this process, the 3-aminopropyltriethoxysilane (APTES) was bonded onto the surface of the substrate 6, and moreover an excessive coupling reaction was brought about, thus bonding molecules of the coupling agent together end-to-end (i.e. the APTES was formed on the surface of the photocatalyst layer in a multilayer-adsorbed state).

After the refluxing, the substrate was washed a plurality of times with an alcohol such as methanol and a 0.1 M potassium phosphate buffer. After that, glutaraldehyde (see reference numeral 12 in the drawings) was added to a concentration of 3.0 wt% into the buffer. After that, a similar process to in the case of manufacturing the monolayer-bridged photocatalytic material described earlier was carried out, thus obtaining the desired multilayer-bridged photocatalytic material. It was verified that the thickness of the bridging parts of the multilayer-bridged photocatalytic material obtained was higher than that of the monolayer-form bridging parts of the monolayer-bridged photocatalytic material through surface analysis of the photocatalyst layer using an atomic force microscope (AFM). This means that the distance between the photocatalyst layer and the CD4 holding substance will be greater than in the case of the monolayer-bridged photocatalytic material.

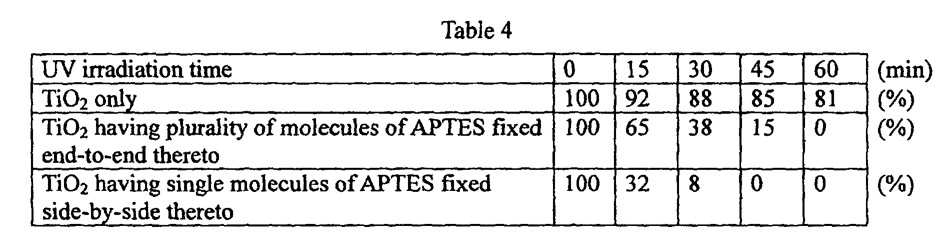

As shown in Table 4 and FIG. 13, it was found that the HIV inactivation efficiency relative to the UV irradiation time is higher for the monolayer-bridged photocatalytic material than for the photocatalytic material not having bridging molecules and a holding substance or the multilayer-bridged photocatalytic material.

With the monolayer-bridged photocatalytic material, the thickness of the layer of bridging molecules is as low as possible, and hence the irradiation of ultraviolet radiation onto the titanium dioxide film is not prone to being impeded by the bridging molecules. Furthermore, the distance between the titanium dioxide film and the CD4 is low. The HIV held by the CD4 can thus be inactivated (made harmless) efficiently by photocatalysis occurring in the vicinity of the titanium dioxide film. Moreover, excessive bonding of the glutaraldehyde to the silane coupling agent is also suppressed, and hence the bridging molecule film thickness can be made approximately uniform over the whole of the material.

Note that, in this test example, the HIV solution that was the sample to be treated was made to come into contact with the photocatalytic material, and in this state treatment in which the harmful substance (HIV) held by the holding substance (CD4) is inactivated while irradiating with light was carried out continuously; however, there is no limitation to this mode of use. For example, first, without carrying out irradiation with light, treatment may be carried out in which the sample to be treated is made to come into contact with the photocatalytic material, thus holding the harmful substance on the holding substance. Next, after the contact between the sample to be treated and the photocatalytic material has been completed (i.e. the photocatalytic material has been separated out from the sample to be treated), treatment may be carried out in which the photocatalytic material is irradiated with light to inactivate the harmful substance that has been held (captured) in advance by the holding substance. With this step-like approach, denaturation of constituent components (useful components) in the sample to be treated by the photocatalysis can be reliably prevented, and moreover parts (fragments) of the harmful substance produced through decomposition by the photocatalysis can be reliably prevented from contaminating the treated sample.

### (Test Example 2)

A photocatalytic material was manufactured in which a titanium dioxide film, bridging molecules and a holding substance were introduced onto the inner surface of a cylindrical tubular substrate, and HIV inactivation treatment was carried out as before.

Specifically, an optically transparent quartz glass tube of inside diameter (φ) 2 mm, thickness 0.5 mm and length 200 mm was taken as a substrate, and a commercially sold TiO₂ coating liquid (ST-K01 made by Ishihara Sangyo Kaisha, Ltd.) was applied onto the inner surface of the substrate, and then the substrate was held at 500°C in atmospheric air for 30 minutes, thus baking a titanium dioxide film onto the inner surface of the tubular substrate.

Next, a vapor of a toluene solvent containing 3-aminopropyltriethoxysilane was fed into the tubular substrate from a tube that had been connected to the tubular substrate in advance, thus arranging molecules of the 3-aminopropyltriethoxysilane in the form of a monolayer on the surface of the titanium dioxide film as with the monolayer-bridged photocatalytic material described earlier.

Next, washing and drying, and then treatment to introduce glutaraldehyde were carried out as in the case of manufacturing the monolayer-bridged photocatalytic material described earlier, thus forming monolayer-form bridging molecules on the inside of the tube. It was verified that the bridging molecules formed on the inner surface of the tube were in a monolayer state by measuring the film thickness of the 3-aminopropyltriethoxysilane fixed to the surface of the titanium dioxide film using spectral ellipsometry. Specifically, the measured value of this film thickness was 1.1 ± 0.2 nm.

Next, treatment to fix on CD4 was carried out as in the case of manufacturing the monolayer-bridged photocatalytic material described earlier, thus obtaining a tubular photocatalytic material.

A sterilized disposable tube was attached to one end of the tubular photocatalytic material, and a sample containing HIV (a liquid sample that had been prepared such that the p24 antigen concentration was 100 ng/ml) was fed into the tube at a rate of 200 ml/hr using an infusion pump. At this time, a culture solution (the previously mentioned RPMI-1640 medium) was filled into the tubular photocatalytic material in advance, thus always keeping the titanium dioxide film and the CD4 inside the quartz glass tube moist. Moreover, a sterilized collecting vessel was installed in advance on the outflow side of the quartz glass tube. During the operation of the infusion pump, the quartz glass tube was continuously irradiated with ultraviolet radiation of wavelength 300 to 400 nm (intensity of ultraviolet radiation: 350 ± 20 µW/cm²). The light source was the same black light as that used in Test Example 1.

30 minutes after commencement of the UV irradiation, ten 500 µl samples were taken from the liquid that had accumulated in the collecting vessel. After that, the ten samples were each mixed into a culture suspension of H9 cells as host cells. Each of the mixtures of the cells and a sample was then cultured for 14 days in an incubator under conditions of 37°C and 5% CO₂. Next, the amount of p24 antigen in the culture solution (supernatant) was measured, thus indirectly measuring the amount of infectious virus remaining after the UV irradiation treatment. The result was that is was found that the HIV had been inactivated in all of the samples.

A photocatalytic material having such a tubular shape can easily be manufactured, and moreover can easily be made light in weight and/or small in size. Such a photocatalytic material can thus easily be used as a bioreactor for inactivating a specific harmful substance, and is highly versatile and practical.

### (Test Example 3)

The monolayer-bridged photocatalytic material and the multilayer-bridged photocatalytic material described earlier were each put into 500 µl of ultrapure water, and irradiation was carried out for 120 minutes with ultraviolet radiation of wavelength 300 to 400 nm and intensity 2000 µW/cm². After that, each sample was removed from the ultrapure water, and immersed in a solution of Coomassie brilliant blue (CBB), which is a protein staining agent.

The result was that the multilayer-bridged photocatalytic material was not stained by the CBB solution, whereas the monolayer-bridged photocatalytic material was stained by the CBB solution. This suggests that even upon irradiating with relatively strong ultraviolet radiation, the CD4 does not break away from the surface of the photocatalyst layer (titanium dioxide film) of the monolayer-bridged photocatalytic material. Moreover, it suggests that with the monolayer-bridged photocatalytic material, the bridging molecules can be arranged densely in the form of a monolayer on the surface of the photocatalyst layer, whereby the CD4 can be held at high density on the surface of the photocatalyst layer.

With the monolayer-bridged photocatalytic material manufactured using the manufacturing process described earlier, as shown in FIG. 1, a 3-aminopropyltriethoxysilane monomolecular film is formed on the surface of the titanium dioxide film 5, and the 3-aminopropyltriethoxysilane and the titanium dioxide film 5 are bound together by Si-O bonds, i.e. inorganic bonds. It can thus be conjectured that there is little effect on the bridging molecules 7 by the photocatalytic action of the titanium dioxide film 5, and that this is why the bridging molecules and the CD4 did not break away from the titanium dioxide film 5.

### (Test Example 4)

The film formation time was suitably changed during the normal pressure CVD used when manufacturing the monolayer-bridged photocatalytic material and the multilayer-bridged photocatalytic material, thus investigating how the thickness of the titanium dioxide film could be controlled.

Specifically, the film formation time for the titanium dioxide film in the normal pressure CVD described earlier was changed within a range of 0.5 to 7.5 minutes.

**Table 5**

| Film formation time (min) | Titanium dioxide film thickness (µm) |
|---|---|
| 0.5 | 0.1 |
| 1.0 | 0.6 |
| 1.5 | 0.9 |
| 2.0 | 1.3 |
| 2.5 | 1.6 |
| 3.0 | 1.7 |
| 3.5 | 2.2 |
| 4.0 | 2.3 |
| 4.5 | 2.9 |
| 5.0 | 3.8 |
| 6.0 | 4.4 |
| 7.5 | 5.0 |

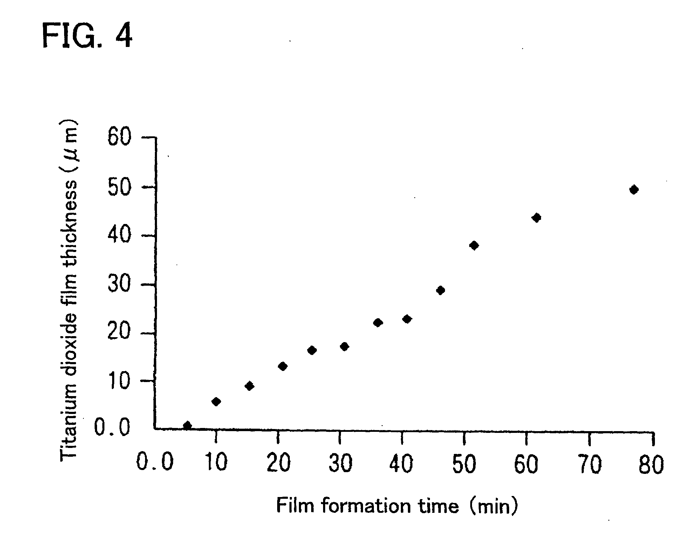

As shown in Table 5 and FIG. 4, it was verified that a relationship of proportionality holds between the film formation time and the thickness of the titanium dioxide film.

### (Test Example 5)

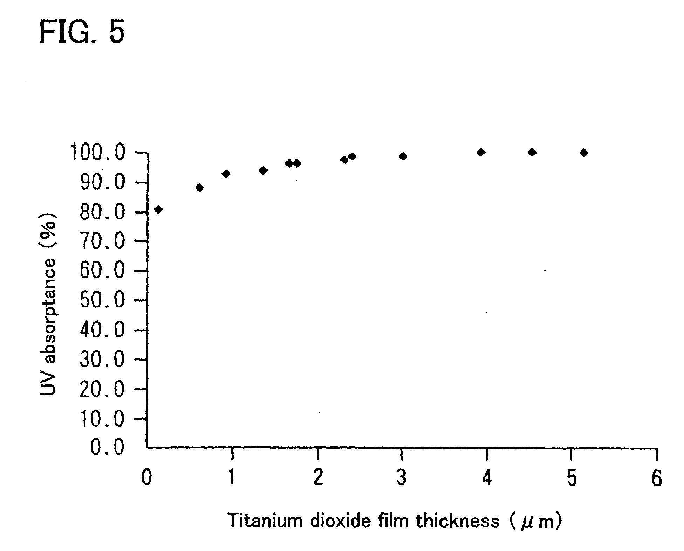

Next, tests were carried out into the relationship between the thickness of the titanium dioxide film and the UV absorptance. Specifically, a process was carried out as in Test Example 4, thus forming a photocatalyst layer (titanium dioxide film) of any of various thicknesses on one surface of a sheet-like silica glass substrate 6 (see FIG 2). Ultraviolet radiation was then irradiated from the side on which the titanium dioxide film had not been formed, i.e. the side of the non-coated surface, and the intensity of the ultraviolet radiation passing through the substrate and the photocatalyst layer was measured, and the UV absorptance of the substrate 6 was determined. The previously mentioned black light made by Toshiba Lighting and Technology Corporation was used as the light source for the ultraviolet radiation, and the intensity of the ultraviolet radiation was measured using an ultraviolet radiation measuring instrument made by Minolta (UM-10 measuring unit, UM-360 light receiver). The results are shown in Table 6 and FIG 5.

**Table 6**

| Titanium dioxide film thickness (µm) | UV absorptance (%) |
|---|---|
| 0 | 5.0 |
| 0.1 | 80.6 |
| 0.6 | 88.0 |
| 0.9 | 92.4 |
| 1.3 | 93.8 |
| 1.6 | 96.5 |
| 1.7 | 96.6 |
| 2.2 | 97.4 |
| 2.3 | 98.1 |
| 2.9 | 98.2 |
| 3.8 | 99.6 |
| 4.4 | 99.7 |
| 5.0 | 99.8 |

From Table 6 and FIG. 5, it was found that the UV absorptance rose slightly upon increasing the thickness of the titanium dioxide film. It was found that in the case that the thickness of the titanium dioxide film was 3.8 µm or more, the UV absorptance was 99% or more (i.e. the transmittance was 1% or less).

It is clear from these results that if the titanium dioxide film is made too thin, then there will be a risk of the UV absorptance dropping too much and hence it not being possible to obtain sufficient photocatalysis. Moreover, it is also possible that a plasma component or the like in the sample being treated could be denatured by ultraviolet radiation of relatively high intensity that has passed through the substrate and the photocatalyst layer. To avoid these problems, it is thus suitable to form the photocatalyst layer (titanium dioxide film) to a thickness such that the UV absorptance is at least 90% (i.e. the transmittance is not more than 10%), and it is preferable to form the photocatalyst layer (titanium dioxide film) to a thickness such that the UV absorptance is at least 99% (i.e. the transmittance is not more than 1%)

### (Test Example 6)

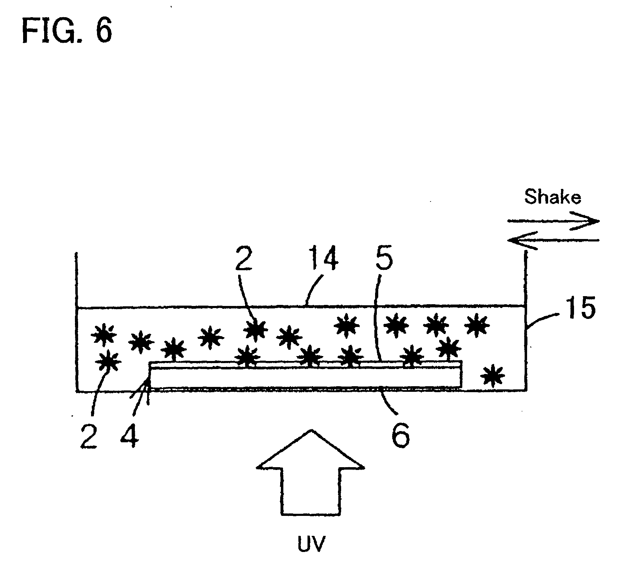

Next, tests were carried out into the relationship between the thickness of the titanium dioxide film possessed by a photocatalytic material and the antibacterial ability against E. coli as a harmful substance. These tests were carried out using photocatalytic materials having photocatalyst layers (titanium dioxide films) of various thicknesses as manufactured in Test Example 5. Specifically, as shown in FIG. 6, 1.0 ml of physiological saline 14 to which E. coli (E. coli K-12) as a harmful substance 2 had been added such that the number of bacteria was approximately 10⁵/ml was put into a polypropylene petri dish 15 (inside diameter 15 mm). The photocatalytic material 4 of one of the film thicknesses was then put into the petri dish 15 with the photocatalyst layer (titanium dioxide film) 5 facing upward, and then, while shaking the petri dish 15, ultraviolet radiation of intensity 400 µW/cm² was immediately irradiated from the side of the non-coated surface of the substrate 6 using a black light (one made by Toshiba Lighting and Technology Corporation as mentioned earlier).

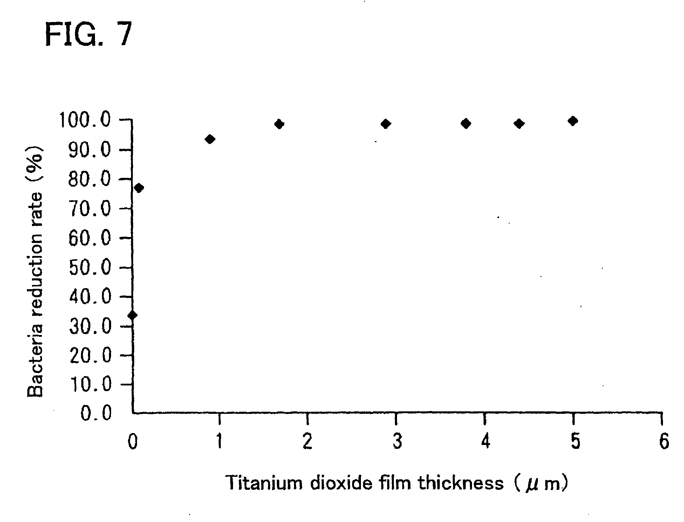

At this time, the UV irradiation time was made to be 0, 30, 60 or 120 minutes. After the UV irradiation had been completed, each of the E. Coli suspensions was inoculated into an ordinary BHI culture medium, and culturing was carried out in an incubator for approximately 16 hours at 37°C. After that, the E. Coli count was taken. At this time, the ratio of the bacterial count for each sample to the bacterial count for the sample for which the UV irradiation was carried out for 0 minutes was taken as the bacteria survival rate (%) for the sample in question. The bacteria reduction rate (sterilization rate) (%) is thus equal to 100 - bacteria survival rate. The results are shown in Table 7 and FIG. 7.

**Table 7**

| Titanium dioxide film thickness (µm) | UV irradiation time (min) | | | |
|---|---|---|---|---|
| | Bacteria survival rate (%) | | | |
| | 0 | 30 | 60 | 120 |
| 0 | 100.0 | 100.0 | 70.7 | 66.3 |
| 0.1 | 100.0 | 77.5 | 57.3 | 22.5 |
| 0.9 | 100.0 | 86.7 | 38.3 | 5.8 |
| 1.7 | 100.0 | 82.3 | 49.2 | 0.8 |
| 2.9 | 100.0 | 72.5 | 59.4 | 1.0 |
| 3.8 | 100.0 | 73.6 | 25.9 | 0.9 |
| 4.4 | 100.0 | 46.4 | 29.0 | 0.7 |
| 5.0 | 100.0 | 78.6 | 18.9 | 0.0 |

As shown in Table 7 and FIG 7, good results were obtained for the bacteria reduction rate with a titanium dioxide film thickness of 0.9 µm or more. Moreover, it was found that the E. Coli were completely killed upon irradiating the photocatalytic material for at least 2 hours with 400 µW/cm² of ultraviolet radiation. Furthermore, it was found that a high bacteria reduction rate of 99% or more was exhibited in the case that the thickness of the titanium dioxide film of the photocatalytic material was 1.7 µm or more.

### (Test Example 7)

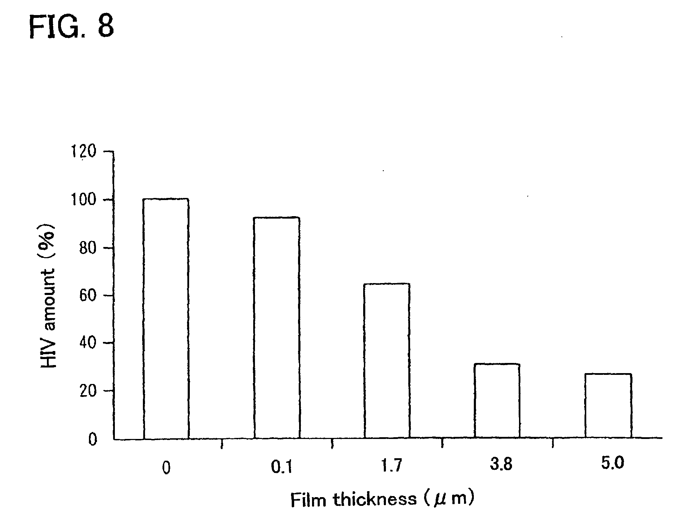

Next, tests were carried out into the relationship between the thickness of the titanium dioxide film possessed by a photocatalytic material and the anti-HIV activity in the case of targeting HIV as a harmful substance. These tests were carried out using photocatalytic materials having photocatalyst layers (titanium dioxide films) of various thicknesses as manufactured in Test Example 5. Specifically, as shown in FIG 6, 1.0 ml of serum 14 to which HIV as a harmful substance 2 had been added such that the p24 antigen concentration was 50 ng/ml was put into a polypropylene petri dish 15 (inside diameter 15 mm) (see FIG. 6). The photocatalytic material 4 of one of the film thicknesses was then put into the petri dish 15 with the photocatalyst layer (titanium dioxide film) 5 facing upward, and then, while shaking the petri dish 15, ultraviolet radiation of intensity 400 µW/cm² was immediately irradiated from the side of the non-coated surface of the substrate 6 using a black light (one made by Toshiba Lighting and Technology Corporation as mentioned earlier). At this time, the UV irradiation time was made to be 30 minutes. After the UV irradiation had been completed, using each of the serum samples, HeLa cells expressing CD4 and CCR5, which are HIV infection receptors, were cultured for 3 days under conditions of 37°C and 5% CO₂. Note that HeLa cells have an expression mechanism for β-gal, which is derived from an HIV promoter, and β-galactosidase is produced in the cells upon infection. The cells thus turn blue upon adding X-gal after the culturing. By counting the number of cells that have turned blue, the virus infection rate can be quantified indirectly. The results are shown in FIG 8. In this bar chart, the ratio of the amount of HIV in each sample liquid to the amount of HIV in the sample liquid treated with the material for which the thickness of the titanium dioxide film was 0 µm (i.e. the material not containing a photocatalyst layer) is shown as a percentage (%).

As is clear from this graph, it was found that, as with the case of E. Coli in Test Example 6 described earlier, the photocatalytic materials used here exhibit anti-viral activity against HIV, i.e. anti-HIV activity. Moreover, as a result of controlling the thickness of the titanium dioxide film of the photocatalytic material, it was found that the most effective anti-HIV ability is exhibited when the thickness of the titanium dioxide film is approximately 5.0µm.

### (Test Example 8)

Next, tests were carried out into the selective anti-HIV activity using photocatalytic materials manufactured by bonding a holding substance onto the surface of a titanium dioxide film via bridging molecules. In these tests, the materials used were obtained by taking photocatalytic materials having photocatalyst layers (titanium dioxide films) of various thicknesses as manufactured in Test Example 5, and forming bridging molecules and a holding substance (CD4) on the surface of the titanium dioxide film using the same manufacturing process as with the multilayer-bridged photocatalytic material described earlier.

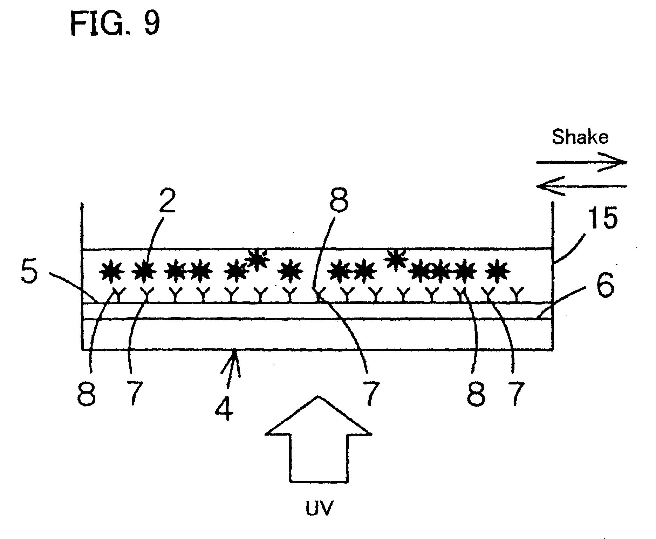

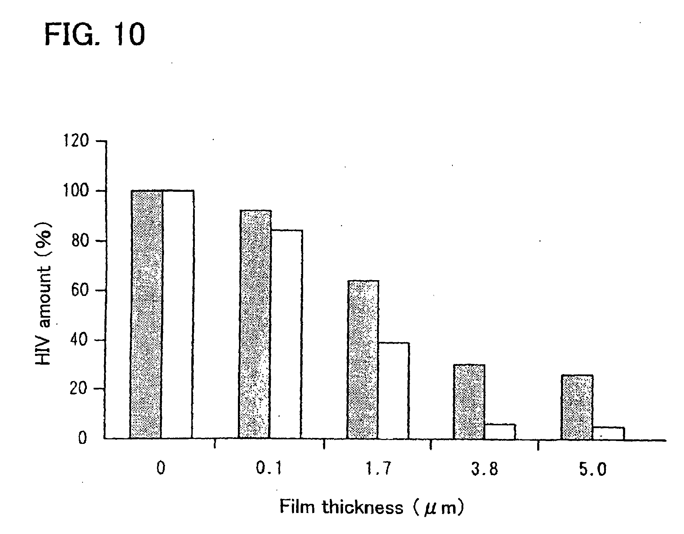

First, as shown in FIG. 9, a disk-shaped photocatalytic material 4 of diameter 15 mm and thickness 0.5 mm in which a holding substance 8 (CD4) had been bonded to the surface of a titanium dioxide film 5 of a prescribed thickness was put into a petri dish 15 (inside diameter 15 mm) with the photocatalyst layer (titanium dioxide film) 5 facing upward. Serum in which the amount of HIV had been adjusted such that the p24 antigen concentration was 50 ng/ml was then added into the petri dish 15 to a depth of approximately 2 mm from the surface of the titanium dioxide film 5. Approximately 3.53 µl of plasma to which heparin, which is an anticoagulant, had been added was then further added. While shaking the petri dish 15, ultraviolet radiation of intensity 400 µW/cm² was then irradiated from the side of the non-coated surface of the substrate 6 using a black light (one made by Toshiba Lighting and Technology Corporation as mentioned earlier). At this time, the UV irradiation time was made to be 30 minutes. After the UV irradiation had been completed, using each of the sample liquids, HeLa cells expressing CD4 and CCR5, which are HIV infection receptors, were cultured for 3 days under conditions of 37°C and 5% CO₂. The results are shown in FIG. 10. In this bar chart, the ratio of the amount ofHIV in each sample liquid to the amount of HIV in the sample liquid treated with the material for which the thickness of the titanium dioxide was 0 µm (i.e. the material not containing a photocatalyst layer) is shown as a percentage (%). Of the pair of bars rising up from each film thickness on the horizontal axis, the shaded bar on the left shows the results for a photocatalytic material not containing the bridging molecules or the holding substance, and the white bar on the right shows the results for a photocatalytic material onto which the bridging molecules and the holding substance were introduced.

As is clear from this bar chart, it was found that, compared with the case that the holding substance was not bonded to the surface of the titanium dioxide film, the anti-HIV activity can be markedly improved by introducing the holding substance. This shows that HIV was selectively taken from the sample liquid and held by the holding substance of the photocatalytic material, and then the held HIV was inactivated and decomposed by the photocatalytic action exhibited by the titanium dioxide film. Moreover, it was found that the anti-HIV activity of the photocatalytic material can be adjusted by suitably changing the thickness of the titanium dioxide film. In particular, it was found that in the case that the thickness of the titanium dioxide film is 1 µm or more (e.g. 1 to 7 µm), particularly preferably 3 µm or more (e.g. 3 to 7 µm), more preferably 5 µm or more (e.g. 5 to 7 µm), high anti-HIV activity can be exhibited.

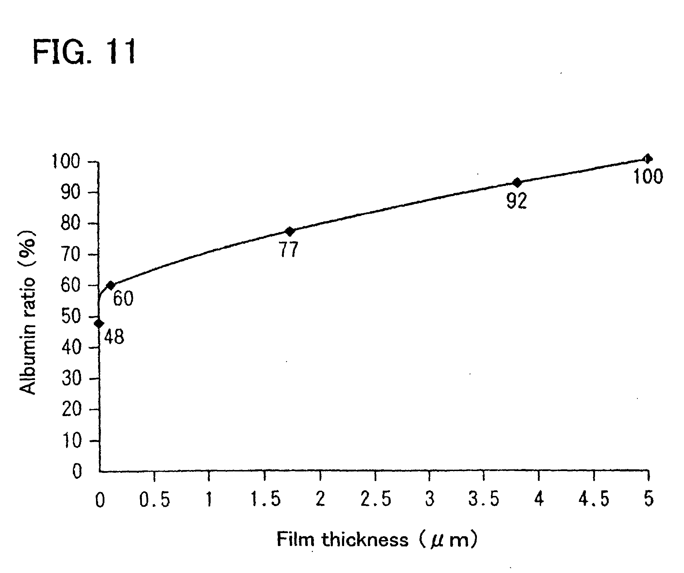

Furthermore, for the tests carried out using the photocatalytic materials onto which the holding substance had been introduced, the change in the amount of albumin (a plasma component) between before and after the test was analyzed using an ELISA (enzyme-linked immunosorbent assay) method as a biochemical technique. The results are shown in FIG. 11. In this graph, the ratio of the amount of albumin remaining in the sample liquid after the UV irradiation to the amount of albumin contained in the sample liquid before the UV irradiation (i.e. the residual ratio) is shown as a percentage (%).

As is clear from this graph, in the case that the thickness of the titanium dioxide film of the photocatalytic material used was low at 0.1 µm, the plasma component (albumin) contained in the sample liquid was denatured by the UV irradiation. However, as the thickness of the titanium dioxide film of the photocatalytic material used increases, the extent of denaturation of the plasma component (albumin) by the UV irradiation drops, and in the case that the film thickness was high at 5.0 µm, the plasma component was substantially not denatured by the UV irradiation. This shows that, because a titanium dioxide film has a high UV absorptance, by making the film thickness high, ultraviolet radiation, which will decompose the plasma component, can be prevented from being irradiated strongly onto the sample liquid itself. Moreover, by introducing the holding substance (CD4), the frequency of the plasma component coming into direct contact with the titanium dioxide film can be reduced.

### (Test Example 9)

Next, the effect of the thickness of the photocatalyst layer (titanium dioxide film) on the strength of attachment of the titanium dioxide film to the substrate (i.e. the resistance to peeling off) was evaluated by carrying out tests in accordance with the cross-cut tape method of JIS K S500 (General Test Methods for Coating Materials 8.5.3).

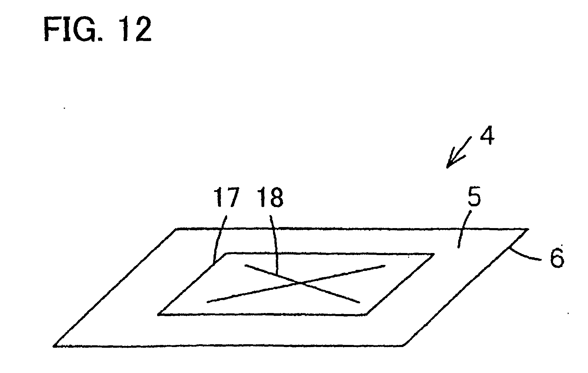

Specifically, as shown in FIG. 12, photocatalytic materials 4 were manufactured by forming a titanium dioxide film 5 of any of various thicknesses on the surface of a silica glass substrate 6 of width 150 mm x length 70 mm x thickness 0.5 mm using normal pressure CVD as in Test Example 4 described earlier. Ordinary cellophane adhesive tape 17 was stuck onto the surface of the titanium dioxide film 5 of each photocatalytic material 4. Next, X-shaped cuts (cross cuts) 18 of length 40 mm intersecting one another at 30° were cut into the surface of the cellophane adhesive tape 17 using a cutting knife, not shown. Next, another piece of cellophane adhesive tape 17 was stuck onto the surface of the cut cellophane adhesive tape 17. After that, the cellophane adhesive tape 17 was peeled off, and the adhesion of the titanium dioxide film 5 to the substrate 6 was measured. The results are shown in Table 8. The evaluation score is in accordance with JIS. For the photocatalytic materials having an evaluation score of 10, the titanium dioxide film did not peel off whatsoever.

**Table 8**

| Titanium dioxide film thickness (µm) | Evaluation score |
|---|---|
| 1 | 10 |
| 2 | 10 |
| 3 | 10 |
| 4 | 10 |
| 5 | 10 |
| 6 | 10 |
| 7 | 4 |
| 8 | 2 |
| 9 | 0 |
| 10 | 0 |

As shown in Table 8, for the titanium dioxide films of thickness up to 6 µm, there was no peeling away from the silica glass substrate. This shows that the titanium dioxide film was vapor-deposited onto the substrate surface sufficiently. From the viewpoint of strength of attachment, the thickness of the photocatalyst layer (here, a film made of a transition metal oxide such as titanium dioxide) formed on the surface of the silica glass substrate is thus preferably approximately 1 to 7 µm, particularly preferably approximately 1 to 6 µm.

Next, a number of preferable embodiments of photocatalytic treatment apparatuses (bioreactors for inactivating a prescribed biologically harmful substance from a liquid or gaseous sample to be treated) provided by the present invention will be described, with reference to the drawings.

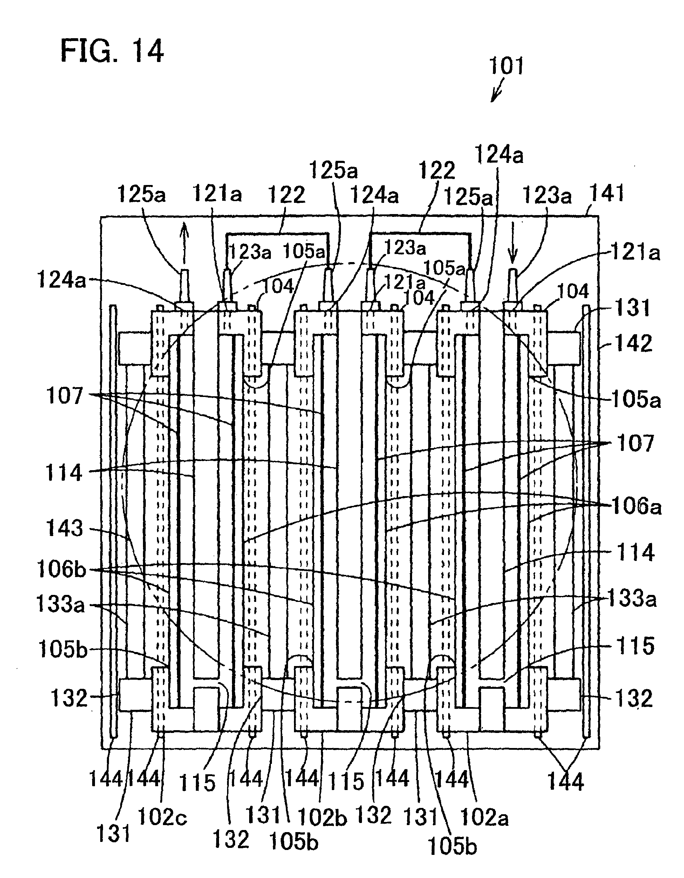

First, with reference to FIGS. 14 to 17, a description will be given of an apparatus 101 characterized by having at least one pair of sheet-shaped optically transparent substrates disposed separated from one another, wherein a space formed between each pair of substrates is used as a flow path along which is fed a fluid to be treated, and further having a wall member disposed in each flow path.

As shown in FIG 14, the photocatalytic treatment apparatus (reactor) 101 comprises a plurality of (here, three) treatment units 102a, 102b and 102c for treating a liquid or gaseous sample to be treated with a photocatalyst. These units 102a, 102b and 102c are housed inside a hollow rectangular parallelepiped-shaped vessel (casing) 142 in a state connected to one another.

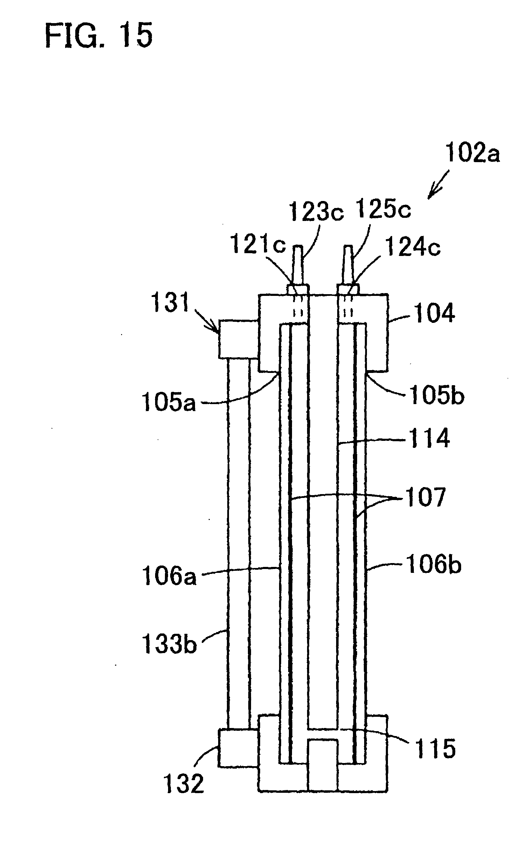

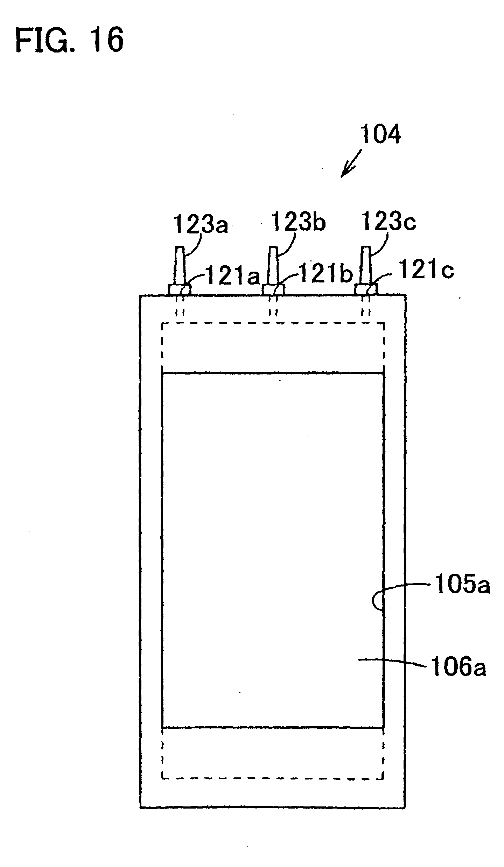

As shown in FIGS. 15 and 16, each of the treatment units 102a, 102b and 102c has a column 104 made of a synthetic resin such as a polycarbonate, this being a long thin hollow rectangular parallelepiped-shaped unit main body (vessel). Rectangular openings 105a and 105b are formed in mutually facing side walls of the column 104. These openings 105a and 105b are blocked respectively with rectangular sheet-shaped transparent substrates 106a and 106b. The transparent substrates 106a and 106b are disposed separated from one another and parallel to one another, i.e. positioned such that the broad surfaces thereof are parallel to one another. The transparent substrates 106a and 106b are made of a material that is able to transmit ultraviolet radiation at high rate (here, quartz glass).

Each of the pair of transparent substrates 106a and 106b that are disposed parallel to one another has formed over approximately the whole of the inner surface thereof a photocatalytic material 107 for holding and inactivating a harmful substance that contaminates or may contaminate a fluid to be treated.

Each photocatalytic material (photocatalyst layer) 107 has a titanium dioxide film formed (coated) over approximately the whole of the inner surface of the transparent substrate 106a or 106b, bridging molecules formed on the surface of the titanium dioxide film, and a holding substance (here, CD4) linked to the titanium dioxide film via the bridging molecules. With the exception of the form of the substrate, this photocatalytic material has the same constitution as the monolayer-bridged photocatalytic material (see FIGS. 1 and 2) described earlier, and can be manufactured through a similar manufacturing process. Redundant repeated description will thus be omitted here.

Moreover, an approximately rectangular plate-shaped wall member 114 made of a synthetic resin is disposed between the pair of transparent substrates 106a and 106b installed in the column 104 of each of the treatment units 102a, 102b and 102c. The wall member 114 is disposed in a state such that the direction in which the surfaces thereof run is aligned with the longitudinal direction of the column 104 (i.e. in a state such that the surfaces of the wall member are parallel to the inner surface of each of the transparent substrates 106a and 106b). The wall member 114 functions as a partitioning plate. The partitioning plate (wall member) 114 is disposed between (i.e. in the space between) the pair of transparent substrates 106a and 106b installed in the column 104, and is opaque, i.e. is able to block light from UV lamps 133a and 133b, described later.

Moreover, end parts in the longitudinal direction of the partitioning plate (wall member) 114 are connected to end parts in the longitudinal direction of the column 104 in a sealed state (with a sealing performance at least such that liquid will not leak out through gaps). Furthermore, one of the end parts in the longitudinal direction of the partitioning plate 114 has provided therein a communicating hole 115 such that a fluid can pass from the portion of the space formed between the pair of transparent substrates 106a and 106b installed in the column 104 on one side of the partitioning plate 114 (i.e. between the transparent substrate 106a and the partitioning plate 114) to the portion of this space on the other side of the partitioning plate 114 (i.e. between the other transparent substrate 106b and the partitioning plate 114). As a result, the communicating hole 115 forms a communicating flow path from the space between the transparent substrate 106a and the partitioning plate 114 to the space between the other transparent substrate 106b and the partitioning plate 114.

A plurality of (e.g. three) inflow ports 121 a, 121b and 121 c are formed in each of the treatment units 102a, 102b and 102c in an upper end part of the column 104 thereof positioned between the transparent substrate 106a and the partitioning plate 114, so that the fluid to be treated can be introduced into the space between the transparent substrate 106a and the partitioning plate 114. These inflow ports 121 a, 121 b and 121c are formed in the upper end part of the column, arranged at equal intervals along the width direction of the column. The inflow ports 121a, 121b and 121c have tapered inflow tubes 123a, 123b and 123c attached thereto. The inflow tubes 123a, 123b and 123c each have connected thereto one end of a long thin hollow cylindrical infusion tube 122 in a sealed state such that liquid will not leak out. The infusion tubes 122 are made of a flexible material such as a polyamide synthetic resin. Tubes of a material widely used in medical equipment for artificial dialysis and so on are suitable.

Moreover, a plurality of (e.g. three) outflow ports 124a and 124c are provided in each of the treatment units 102a, 102b and 102c in the upper end part of the column 104 thereof positioned between the other transparent substrate 106b and the partitioning plate 114, i.e. where the inflow ports 121a, 121b and 121c are not formed, so that the fluid can be discharged out from the space between the transparent substrate 106b and the partitioning plate 114. These outflow ports 124a and 124c are formed in the upper end part of the column, arranged at equal intervals along the width direction of the column 104. The outflow ports 124a and 124c have tapered outflow tubes 125a and 125c attached thereto. The outflow tubes 125a and 125c each have connected thereto one end of one of the infusion tubes 122 in a sealed state such that liquid will not leak out.

As a result of the above constitution, the fluid to be treated is introduced into the space between the transparent substrate 106a and the partitioning plate 114 from the infusion tubes 122 connected to the inflow tubes 123a, 123b and 123c, flows through the space between the transparent substrate 106a and the partitioning plate 114, passes through the communicating hole 115, and flows into the space between the other transparent substrate 106b and the partitioning plate 114. The fluid then flows through the space between the transparent substrate 106b and the partitioning plate 114, and is discharged out into infusion tubes 122 via the outflow tubes 125a and 125c.

Moreover, for each of the treatment units 102a, 102b and 102c, a UV lamp unit 131 is installed on a side surface in the thickness (width) direction of the column 104 (specifically, the surface facing the transparent substrate 106a), this being a light source unit for irradiating ultraviolet radiation onto the titanium dioxide film 108 via the transparent substrate 106a.

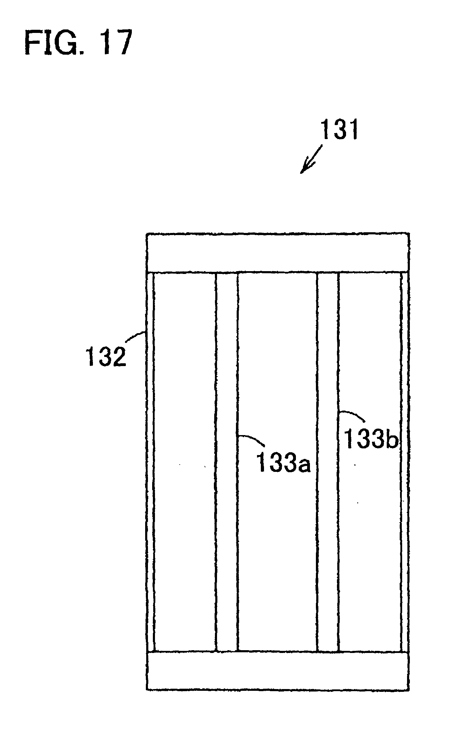

As shown in FIG. 17, each UV lamp unit 131 has a rectangular frame-shaped synthetic resin frame 132. The external dimension in the longitudinal direction of the frame 132 is shorter than the external dimension in the longitudinal direction of the column 104. Moreover, the internal dimension in the longitudinal direction of the frame 132 is preferably equal to or longer than the longitudinal dimension of the transparent substrates 106a and 106b. The external dimension in the width direction of the frame 132 is approximately equal to the external dimension in the width direction of the column 104. The internal dimension in the width direction of the frame 132 is longer than the width dimension of the transparent substrates 106a and 106b.

A plurality of (e.g. two) cylindrical UV lamps 133a and 133b are installed, as light source that irradiates light such as ultraviolet radiation, between inner edges of the two ends in the longitudinal direction of the frame 132, this being in a state bridged across the upper end of the frame. As shown in FIG. 17, end parts of each of the UV lamps 133a and 133b are electrically connected to the inner edges of the two ends in the longitudinal direction of the frame 132. Moreover, the UV lamps 133a and 133b are disposed side-by-side in the width direction of the frame 132 separated from one another (i.e. with a space therebetween).

As the UV lamps 133a and 133b used in the apparatus 101, a black light that emits light of wavelength 300 to 400 nm, a low-pressure mercury lamp that emits ultraviolet radiation with a peak wavelength around 254 nm (e.g. an HL lamp made by Noritake Company Limited), or the like is suitable. A fluorescent lamp or the like that emits visible light of peak wavelength approximately 600 nm can also be used.

Moreover, to prevent the temperature of the fluid passing through the treatment units 102a, 102b and 102c rising due to heat discharged from the UV lamps 133a and 133b, the treatment apparatus 101 is provided with cooling means (a cooling unit) 141. The cooling unit 141 is constituted substantially from the vessel 142, and a fan 143 that is provided in an opening in part of the vessel 142 as blowing means for introducing external air into the vessel 142. As shown in FIG. 14, the fan 143 is disposed such that cooling air can pass efficiently between the UV lamps 133a and 133b and the transparent substrates 106a and 106b inside the vessel 142. Specifically, in a state in which the treatment units 102a, 102b and 102c have been housed in the vessel 142, the fan 143 is positioned in the direction of a side surface of each of the treatment units 102a, 102b and 102c (i.e. in the direction of an edge surface of each of the sheet-shaped transparent substrates 106a and 106b). Moreover, exhaust openings 144 for exhausting, to the outside, air that has been blown into the vessel 142 by the fan 143 are provided in a side surface of the vessel 142 facing the fan 143. In the apparatus according to the present embodiment, a plurality of (eight) slit-shaped exhaust openings 144 are provided. When the treatment units 102a, 102b and 102c have been housed in prescribed positions in the vessel 142, looking from the direction in which the fan 143 is disposed, the exhaust openings 144 are formed so as to be positioned between the UV lamps 133a and 133b and the transparent substrates 106a and 106b of the treatment units 102a, 102b and 102c (see FIG. 14).

Next, a description will be given of the assembly of the treatment apparatus 101 according to the present embodiment having the constitution described above. First, the side of the second treatment unit 102b on which a UV lamp unit 131 has been installed is made to come into contact with and is connected to the side of the first treatment unit 102a on which a UV lamp unit 131 has not been installed. Moreover, the side of the third treatment unit 102c on which a UV lamp unit 131 has been installed is made to come into contact with and is connected to the side of the second treatment unit 102b on which a UV lamp unit 131 has not been installed. Next, another UV lamp unit 131 of the same form is also made to come into contact with and is connected to the side of the third treatment unit 102c on which a UV lamp unit 131 has not been installed.

Next, as shown in FIG. 14, the outflow tube 125a of the first treatment unit 102a and the inflow tube 123a of the second treatment unit 102b are connected together using a infusion tube 122 (made by Terumo Corporation). The outflow tube 125a of the second treatment unit 102b and the inflow tube 123a of the third treatment unit 102c are similarly connected together using a infusion tube 122. As a result, the flow paths (i.e. the spaces between the pairs of transparent substrates 106a and 106b) of the three treatment units 102a, 102b and 102c are connected together in series. Moreover, the setup is made to be such that the distance from the outer surface of the UV lamps 133a and 133b installed in each UV lamp unit 131 to the nearest transparent substrate 106a or 106b of a treatment unit 102a, 102b or 102c is approximately 10 mm. The intensity of the ultraviolet radiation due to the UV lamp 133a or 133b at the outer surface of each transparent substrate 106a or 106b of each treatment unit 102a, 102b or 102c was measured to be 3000 µW/cm² (result of measurement using the ultraviolet radiation intensity meter made by Minolta mentioned earlier).

The three treatment units 102a, 102b and 102c for which the flow paths have been connected together in series and the UV lamp units 131 are then installed in a vessel (casing) 142 having a fan 143 provided therein as described above, thus constructing the treatment apparatus (reactor) 101 according to the present embodiment.

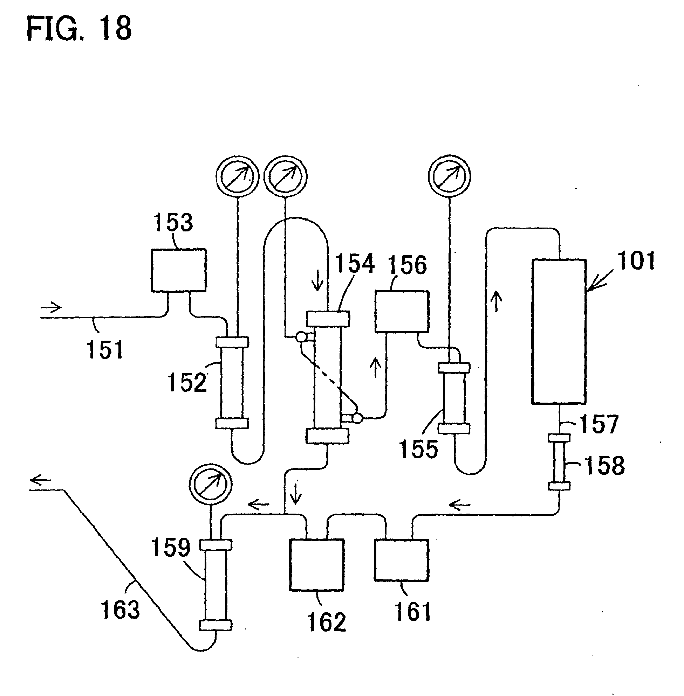

Next, a description will be given of a preferable mode of use of this apparatus (reactor) 101. FIG 18 shows a blood treatment system having the apparatus 101 incorporated therein. A blood sample collected from a vein or the like of a person, not shown, is fed into a infusion tube 151. A pump 153 and a pressure measuring instrument (or flow meter) 152 are connected to the tube 151, and hence by operating the pump 153 while measuring the pressure or flow rate of the blood sample (fluid) flowing through the tube 151, the pressure (flow rate) of the blood sample (fluid) flowing through the infusion tube 151 can be regulated. The pump 153 is operated, whereby blood at a prescribed pressure (flow rate) is fed into a plasma separator 154. The blood sample is fractionated into a plasma component and a corpuscle component in the plasma separator 154, with an operator measuring the pressure before and after the separation of the blood during this.

The plasma obtained through the separation in the plasma separator 154 is then regulated to a prescribed pressure (flow rate) using a pump 156 and a pressure measuring instrument (or flow meter) 155 like those mentioned above, and is then introduced into the photocatalytic treatment apparatus 101 according to the present embodiment.

Specifically, the UV lamps 133a and 133b of the treatment apparatus 101 are lit, and the fan 143 of the cooling unit 141 is operated, and then in this state, the plasma is fed into the flow path in the first treatment unit 102a (i.e. the space between one of the pair of transparent substrates 106a and 106b, i.e. the transparent substrate 106a, and the partitioning plate 114) via the inflow tubes 123a, 123b and 123c of the first treatment unit 102a, which has been connected to the tube 151. The plasma that has been fed in passes through the communicating hole 115, flows into the flow path on the other side of the partitioning plate 114 (i.e. the space between the other one of the pair of transparent substrates 106a and 106b, i.e. the transparent substrate 106b, and the partitioning plate 114), and is then fed from the outflow tubes 125a and 125c through the infusion tubes 122 and then the inflow tubes 123a, 123b and 123c of the second treatment unit 102b and into the flow path in the second treatment unit 102b (see FIG. 14). As with the first treatment unit 102a, the plasma then flows through the flow path of the unit, and is fed out into the infusion tubes 122 from the outflow tubes 125a and 125c. Next, the plasma is fed into the flow path of the third treatment unit 102c via the inflow tubes 123a, 123b and 123c of the third treatment unit 102c (see FIG. 14). As with the first and second treatment units 102a and 102b, the plasma then flows through the flow path of the unit, and is then discharged out of the apparatus 101 through a infusion tube 157 connected to the outflow tubes 125a and 125c.

While the plasma is passing through the three treatment units 102a, 102b and 102c in the apparatus 101 as described above, a specific harmful substance (here, an HIV-originating substance that binds specifically to CD4) is held by the holding substance (CD4). Moreover, the held harmful substance is inactivated by ultraviolet radiation irradiated from the UV lamps 133a and 133b onto the photocatalyst layers (titanium dioxide films) via the transparent substrates 106a and 106b.

The plasma that has been treated by the apparatus 101 according to the present embodiment in this way is then fed to a filter 158 via the infusion tube 157. This filter 158 filters out foreign matter (e.g. holding substance that has broken away form the transparent substrates) contained in the plasma.

After passing through the filter 158, the plasma passes through a heater and is heated to a prescribed temperature, and is mixed with the previously mentioned corpuscle component obtained through the fractionation, and then the mixture is fed to a target supply destination via a infusion tube 163. Moreover, the plasma and corpuscles flowing through the infusion tube 163 (i.e. the blood sample after the harmful substance has been removed and inactivated) may be regulated to a prescribed pressure (flow rate) using a pump 161 and a pressure measuring instrument (or flow meter) 159 like those mentioned above.

As described above, if the present treatment system is used, then, for example, it is possible to separate a harmful substance from plasma obtained by fractionating blood collected from a vein of a person and inactivate the harmful substance, and then return the treated plasma together with the corpuscle component back into a vein of the person.

Using the treatment apparatus 101 according to the present embodiment, HIV inactivation treatment was carried out using a similar procedure to in Test Example 2 described earlier. Note, however, that the p24 antigen concentration of the solution (liquid sample) containing HIV was made to be 50 ng/ml, and this HIV solution was fed into the treatment apparatus at a rate of 100 ml/hr using an infusion pump (made by Terumo Corporation). 30 minutes after commencement of the UV irradiation, ten 500 µl samples were taken from the liquid that had accumulated in the collecting vessel, and evaluation was carried out as in Test Example 2 described earlier; the result was that the HIV had been inactivated in all of the samples.

As described above, with the treatment apparatus 101 according to the present embodiment, UV lamp units 131 are disposed close to the transparent substrates 106a and 106b between the transparent substrates 106a and 106b of the treatment units 102a, 102b and 102c, and moreover a UV lamp unit 131 is disposed on the outside of each of the first and third treatment units 102a and 102c positioned on the two sides of the treatment apparatus 101 close to that treatment unit; as a result, by lighting the plurality of UV lamps 133a and 133b possessed by each of the plurality of UV lamp units 131, the light from the UV lamp units is irradiated uniformly onto the titanium dioxide films formed on the transparent substrates 106a and 106b close to the UV lamps 133a and 133b. The photocatalytic treatment by the titanium dioxide films of the treatment units 102a, 102b and 102c can thus be carried out efficiently. Moreover, with the treatment apparatus 101, because the transparent substrates 106a and 106b have a thin sheet-like shape, the apparatus can be kept compact even if a plurality of treatment units are connected together.

Moreover, because the partitioning plate 114 of each of the treatment units 102a, 102b and 102c does not transmit light, the light from the UV lamps 133a and 133b (in particular, harmful ultraviolet radiation) will not be irradiated excessively onto the sample to be treated such as blood or plasma flowing through the flow paths in the treatment units. Denaturation of components (e.g. blood components or a plasma component) contained in the sample to be treated by the light from the UV lamps 133a and 133b can thus be suppressed.

Furthermore, because the outflow tubes 125a and 125c and the inflow tubes 123a, 123b and 123c of the treatment units 102a, 102b and 102c are connected together by infusion tubes 122, the flow path over which the fluid is treated can be made relatively long even in the case of a compact apparatus. The harmful substance in the fluid introduced into the treatment apparatus can thus be held by the holding substance on the photocatalyst layers more reliably, and hence the rate of inactivation through the photocatalytic action of the titanium dioxide films can be improved.

Furthermore, because, as shown in FIG. 14, a plurality of inflow tubes 123a, 123b and 123c and outflow tubes 125a and 125c are provided in an upper end part of each of the treatment units 102a, 102b and 102c separated from one another at equal intervals along the width direction of the column 104, turbulence that may arise when the fluid is introduced into the column 104 from the inflow tubes 123a, 123b and 123c, and turbulence that may arise when the fluid is discharged to the outside via the outflow tubes 125a and 125c can be prevented. The sample to be treated which is fed continuously into the treatment units 102a, 102b and 102c can thus be treated more uniformly and reliably.

Moreover, because the exhaust openings 144 are provided in the positions described above, the UV lamps 133a and 133b can be cooled efficiently by air blown by the fan 143.

With the apparatus 101 shown in FIGS. 14 to 17, the three treatment units 102a, 102b and 102c were connected together in series such that the fluid flows through the units continuously, but depending on the sample to be treated, the plurality of treatment units 102a, 102b and 102c may instead be disposed in parallel to one another. In the case of connecting several treatment units 102a, 102b and 102c together in parallel, the amount of fluid treated in a certain time can be increased compared with the case that treatment units are connected together in series.

Moreover, the number of treatment units 102a, 102b and 102c constituting the apparatus 101 is not limited to three, but rather more treatment units than this may be provided in series or parallel with one another as required.

Moreover, the cooling unit 141 may have any constitution, so long as the cooling unit 141 is able to prevent an inappropriate rise in the temperature of the sample to be treated (particularly in the case of a sample that readily undergoes thermal denaturation such as blood) due to heat discharged from the UV lamps 133a and 133b.

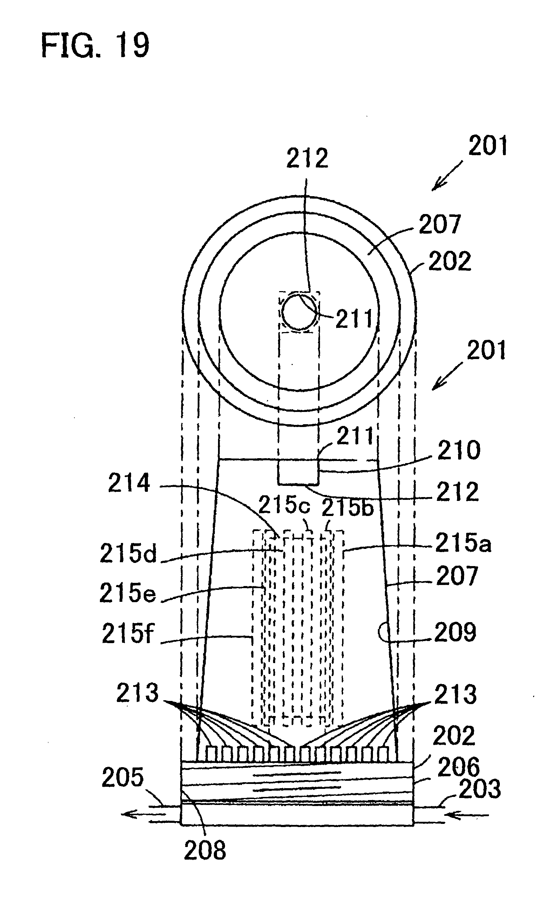

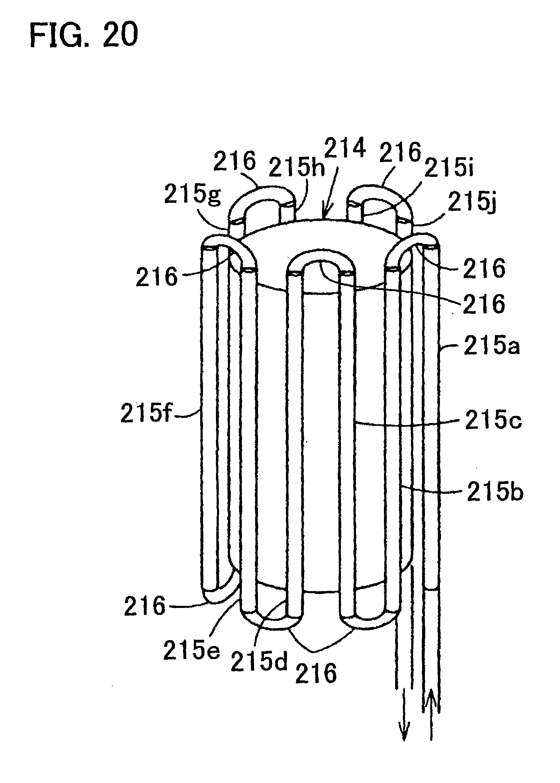

Next, with reference to FIGS. 19 and 20, a description will be given of an apparatus 201 characterized by having a vessel having an optically reflective inner surface, optically transparent substrates disposed inside the vessel, each substrate being tubular and having formed therein a flow path through which a fluid to be treated can flow, and a light source.

As shown in FIG 19, the photocatalytic treatment apparatus (reactor) 201 has a disk-shaped mounting stage 202, and a cover 207 which is a tubular vessel (casing) having a diameter that decreases gradually from a lower end to an upper end thereof. A hollow cylindrical inflow tube 203 through which a fluid (liquid or gas) to be treated is introduced from the outside is provided so as to project out from an outer peripheral surface of the mounting stage 202. Furthermore, an outflow tube 205 through which the fluid is discharged to the outside is provided so as to project out from the outer peripheral surface of the mounting stage 202 opposite the inflow tube 203. Moreover, a thread groove part 206 is formed along the circumferential direction of the outer peripheral surface of the mounting stage 202 on the outer peripheral surface of the mounting stage 202 above the positions of installation of the inflow tube 203 and the outflow tube 205. A thread groove part 208 formed on an inner peripheral surface at a lower end part of the cover 207 is screwed into the thread groove part 206. The cover 207 is preferably formed from stainless steel or a synthetic resin such as a polycarbonate. The inside of each of an upper end surface and a side surface of the cover 207 is mirror-finished by being plated with a metal such as silver (Ag), thus forming a mirrored part 209. This mirrored part 209 is capable of reflecting ultraviolet radiation well.

Moreover, the cover 207 has provided therein a cooling unit 210, which is cooling means for cooling the inside of the cover 207. The cooling unit 210 has an exhaust port 211 that is formed in a central region of an upper end part of the cover 207 and has an axial direction coinciding with the axial direction of the cover 207. The exhaust port 211 has installed therein a fan 212 which is a blowing unit for exhausting air in the cover 207 to the outside.

Furthermore, a plurality of rectangular air intake holes 213, which are vents through which air is introduced into the cover 207 from the outside when the fan 212 is driven, are formed in a lower end region of a peripheral surface part of the cover 207 above the thread groove part 208. These air intake holes 213 are formed at equal intervals along the outer peripheral surface of the cover 207. Typically, the air intake holes 213 are provided around the whole of the outer peripheral surface of the cover 207.

As a result, when the fan 212 is operated in a state in which the thread groove part 208 of the cover 207 has been screwed into the thread groove part 206 of the mounting stage 202, external air is sucked into the cover 207 from the air intake holes 213 of the cover 207. At the same time, air inside the cover 207 is exhausted to the outside from the exhaust port 211. Through the exhaust port 211, the fan 212 and the air intake holes 213, rising of the temperature inside the cover 207 can thus be suppressed, i.e. the inside of the cover 207 can be cooled.

An approximately cylindrical light source 214 is installed in a central region on an upper surface of the mounting stage 202, with the axial direction of the light source 214 aligned in the vertical direction. The outside diameter of the light source 214 is smaller than the outside diameter of the mounting stage 202, and moreover is smaller than the inside diameter of the cover 207 in an upper end region of the cover 207. Moreover, the height of the light source 214 in the axial direction is stipulated to be such that the light source 214 can be disposed inside the cover 207 in the state in which the thread groove part 208 of the cover 207 has been screwed into the thread groove part 206 of the mounting stage 202.

The light source 214 is constituted from a plurality of UV lamps, not shown. Suitable lamps are black lights that emit light of wavelength 300 to 400 nm (e.g. 'FL6BL-B' lamps made by Toshiba Lighting and Technology Corporation), low-pressure mercury lamps that emit ultraviolet radiation with a peak wavelength around 254 nm, and so on. Fluorescent lamps or the like that emit visible light of peak wavelength approximately 600 nm can also be used. Note that with light in a wavelength region from visible light to infrared radiation and beyond, the photocatalyst such as titanium dioxide will not be optically excited, and conversely if the wavelength is too short then constituent components of the fluid to be treated may be denatured or the photocatalytic material may be damaged; UV lamps having a peak wavelength in the region from visible light to ultraviolet radiation, i.e. approximately 150 nm to approximately 600 nm, are thus preferable.

Furthermore, as shown in FIG. 20, a plurality of (e.g. ten) columns (corresponding to substrates) 215a to 215j each formed in a long thin hollow cylindrical shape from an optically transparent material that transmits ultraviolet radiation relatively well such as quartz glass are detachably installed with the axial directions thereof aligned in the vertical direction inside the cover 207 on an outer peripheral region of the light source 214 installed on the upper surface of the mounting stage 202. Each of the columns 215a to 215j has formed over approximately the whole of an inner peripheral surface thereof a photocatalytic material for holding and inactivating a harmful substance that contaminates or may contaminate the fluid to be treated. The photocatalytic material (photocatalyst layer) has a titanium dioxide film formed (coated) over approximately the whole of the inner peripheral surface of the one of the columns 215a to 215j in question, bridging molecules formed on the surface of the titanium dioxide film, and a holding substance (here, CD4) linked to the titanium dioxide film via the bridging molecules. With the exception of the form of the substrate, this photocatalytic material has the same constitution as the monolayer-bridged photocatalytic material (see FIGS. 1 and 2) described earlier, and can be manufactured through a similar manufacturing process.

For example, columns 215a to 215j of inside diameter 2 mm, outside diameter 4 mm and length 150 mm are manufactured from quartz glass using an ordinary method. Titanium tetraisopropoxide is dissolved to a concentration of 0.5 mol/l in ethanol, and then diethanolamine is added in a molar ratio of 1:2 to the titanium tetraisopropoxide, and mixing is carried out to form a uniform solution. Distilled water is then added in an equimolar amount to the titanium tetraisopropoxide, and thorough stirring is carried out, thus producing a coating liquid for forming titanium dioxide films.

This coating liquid is sucked in from one end of each of the columns 215a to 215j and thus introduced into the column, whereby the coating liquid is attached to only the inner peripheral surface of each of the columns 215a to 215j. Each of the columns 215a to 215j is then dried at 100°C, and then baked for 1 hour at 500°C in an air atmosphere. As a result, a thin film made of anatase-type titanium dioxide (the structure of the titanium dioxide can be analyzed by measurement using a thin film X-ray diffraction method) can be formed on the inner peripheral surface of each of the columns 215a to 215j. After that, the bridging molecules and the holding substance (here, CD4) can be introduced onto the surface of the titanium dioxide film using a similar manufacturing process to in the case of manufacturing the monolayer-bridged photocatalytic material (see FIGS. 1 and 2) described earlier. Redundant repeated description will thus be omitted here.

The columns 215a to 215j obtained as described above are detachably attached to a jig, not shown, using clips, not shown, and the jig is fixed to the mounting stage 202 using a method such that attachment/detachment is easy. The columns 215a to 215j are disposed at equal intervals from one another along the circumferential direction of the light source 214, with each of the columns 215a to 215j being separated from the light source 214 by the same distance, for example approximately 15mm. As a result, the columns 215a, 215b, ..., 215j are disposed in a state covering the outer peripheral region of the light source 214 or surrounding the light source 214.

A lower end part of one column 215a is communicatively connected to an end part inside the inflow tube 203 of the mounting stage 202 via a flexible long thin hollow cylindrical infusion tube 216 as piping. The infusion tube 216 is formed from a flexible material such as a polyamide synthetic resin. A tube of a material widely used in medical equipment for artificial dialysis and so on is suitable.

On the other hand, the other end part (here, the upper end part) of the column 215a is communicatively connected to an upper end part of the column 215b adjacent the column 215a via another infusion tube 216. Similarly, as shown in FIG. 20, end parts of other adjacent columns are communicatively connected together via infusion tubes 216 such that all of the columns 215a to 215j around the light source 214 are connected together and form a continuous flow path. One end part of the column 215j (i.e. one end part of the series of columns connected together via the infusion tubes 216) is then communicatively connected to an end part inside the outflow tube 205 via a similar infusion tube 216.

In this way, the columns 215a to 215j are connected together in series, and hence a fluid introduced from the inflow tube 203 flows through the insides (flow paths) of all of the columns 215a to 215j in order, before being discharged from the outflow tube 205.

The treatment apparatus 201 having the above constitution can be used in a similar way to the treatment apparatus 101 shown in FIG. 14. For example, regarding the blood treatment system shown in FIG. 18, the treatment apparatus 201 having the above constitution can be similarly used instead of the apparatus 101 described earlier shown by reference numeral 101. That is, if plasma is fed into the apparatus 201 from the inflow tube 203, then the plasma passes through the columns 215a to 215j, and at this time a harmful substance contained in the plasma is adsorbed (bound) onto and thus held by the holding substance provided on the inner peripheral surfaces of the columns 215a to 215j. The held harmful substance is then inactivated by ultraviolet radiation irradiated from the light source 214 onto the photocatalyst layer (titanium dioxide film) via the quartz glass constituting each of the columns.

Using the treatment apparatus 201 according to the present embodiment, HIV inactivation treatment was carried out using a similar procedure to in Test Example 2 described earlier. Note, however, that the p24 antigen concentration of the solution (liquid sample) containing HIV was made to be 50 ng/ml, and this HIV solution was fed into the treatment apparatus 201 at a rate of 100 ml/hr using an infusion pump (made by Terumo Corporation). 30 minutes after commencement of the UV irradiation, ten 500 µl samples were taken from the liquid that had been discharged from the outflow tube 205 and had accumulated in a collecting vessel, not shown, and evaluation was carried out as in Test Example 2 described earlier; the result was that the HIV had been inactivated in all of the samples.

Next, the cooling efficiency of the apparatus 201 was studied. First, a total of six columns 215a to 215f were disposed at equal intervals around the outer peripheral region of a light source 214. These columns 215a to 215f were then connected together using infusion tubes (made by Terumo Corporation) 216 as described above. Black lights were used as the UV lamps of the light source 214, and the distance from the outer surfaces of each of the black lights to the outer surface of each of the columns 215a to 215f was made to be 15 mm. In this state, the intensity of the ultraviolet radiation at the outer surface of each of the columns 215a to 215f was measured to be 1200 µW/cm² (result of measurement using the ultraviolet radiation intensity meter made by Minolta mentioned earlier). Note that the cover 207 used was made of a polycarbonate, and the inner surface of the cover 207 was plated with silver to form a mirrored part as described earlier.

A small fan 212 was then installed in the exhaust port 211 in the upper end region of the cover 207 (FIG 19), and external air was introduced into the cover 207 from the air intake holes 213 in the lower end region of the cover 207, thus suppressing rising of the temperature inside the cover 207 due to heat discharged from the filaments of the black lights. For example, in the case that the outside air temperature was 25°C, even when the black lights were lit for 1 hour such as to maintain the above-mentioned ultraviolet radiation intensity, the temperature inside the cover 207 could be kept 32°C or lower.

As described in detail above, with the treatment apparatus 201 of the present embodiment, the inner surface of the cover 207 is mirrored, whereby not only light from the light source 214 passes through each of the columns 215a to 21 Sj and is irradiated onto the titanium dioxide films, but also light reflected from the inner surface of the cover 207 passes through each of the columns 215a to 215j and is irradiated onto the titanium dioxide films (photocatalyst layers). As a result, most of the light from the UV lamps of the light source 214 can be irradiated onto the titanium dioxide films of the columns 215a to 215j. The amount of irradiation of light from the light source 214 can thus be reduced, and hence the amount of heat discharged from the light source 214 can be reduced.

Moreover, by surrounding or covering the outer peripheral region of the light source 214 with the plurality of columns 215a to 215j, and making the distance from each of the columns 215a to 215j to the light source 214 be approximately the same for each of the columns 215a to 215j, light from the light source 214 can be irradiated efficiently and uniformly onto each of the titanium dioxide films in the columns 215a to 215j. The photocatalytic treatment using the titanium dioxide films of the columns 215a to 215j can thus be carried out efficiently.

Moreover, because the titanium dioxide film is formed over approximately the whole of the inner surface of each of the columns 215a to 215j, the titanium dioxide film absorbs light from the light source (in particular ultraviolet radiation), and hence the light tends not to be transmitted through into the flow path. Denaturation of components (e.g. blood components or a plasma component) contained in the sample to be treated by the light from the UV lamps of the light source 214 can thus be suppressed.

Furthermore, because the columns 215a to 215j are connected together in series using infusion tubes 216, the flow path over which the fluid is treated and thus the treatment time can be made relatively long even in the case of a compact apparatus. The harmful substance in the fluid introduced into the treatment apparatus can thus be held by the holding substance on the photocatalyst layers more reliably, and hence the rate of inactivation through the photocatalytic action of the titanium dioxide films can be improved. Moreover, because the substrates on each of which the photocatalytic material is formed have a long thin tubular shape in the treatment apparatus 201, the apparatus can be kept compact.

Moreover, with the apparatus 201, because the fan 212 is installed in the exhaust port 211 provided in the upper end region of the cover 207, and the air intake holes 213 are formed in the lower end region of the cover 207, upon driving the fan 212, external air is sucked through the air intake holes 213 into the cover 207, and is then exhausted to the outside through the exhaust port 211. Here, air heated by heat discharged from the UV lamps of the light source 214 will naturally move toward an upper region inside the cover 207 due to the density dropping. The air that has been heated and has risen up through the inside of the cover 207 will then be discharged from the exhaust port 211 at the upper end of the cover 207. With the apparatus 201, efficient cooling of the inside of the cover (vessel) 207 can be realized through this simple constitution.

With the apparatus 201 shown in FIGS. 19 and 20, a total of ten columns 215a to 215j were connected together in series using infusion tubes 216, but depending on the sample to be treated, a plurality of columns of the same form may instead be disposed in parallel to one another. In the case of connecting several columns together in parallel, the amount of fluid treated in a certain time can be increased compared with the case that the columns are connected together in series.

Moreover, the number of treatment units (tubular columns) 215a to 215j constituting the apparatus 201 is not limited to being ten as shown in the drawings, but rather more columns than this may be provided in series or parallel with one another as required.

Moreover, the cooling means (unit) 210 may have any constitution, so long as the cooling means (unit) 210 is able to prevent an inappropriate rise in the temperature of the sample to be treated (particularly in the case of a sample that readily undergoes thermal denaturation such as blood) due to heat discharged from the light source 214.

Concrete examples of the present invention have been described in detail above, but these are merely illustrative, and do not restrict the scope of the claims of the present invention. Any of various modifications or changes to the concrete examples given above are deemed to be included in the art described in the claims.

Moreover, the technical elements described in the present specification and drawings exhibit technical usefulness either alone or in any of various combinations, and there is no limitation to the combinations described in the claims at the time of filing. Moreover, the art illustrated in the present specification and drawings attains a plurality of objects simultaneously, but there is technical usefulness in attaining one of these objects.

## Claims

1. A photocatalytic material used to selectively inactivate a specific biologically harmful substance that may be contained in a liquid or gas to be treated, the material comprising:
a holding substance having holding specificity of selectively holding a specific biologically harmful substance;
a photocatalyst that is able to inactivate the harmful substance held by said holding substance through photocatalysis; and
bridging molecules that link said holding substance to said photocatalyst, said bridging molecules being arranged on the surface of said photocatalyst in the form of a monolayer.

2. The material according to claim 1, wherein said bridging molecules are bonded to the surface of said photocatalyst by inorganic covalent bonds.

3. The material according to claim 1, wherein a layer of thickness 1 to 2 nm constituted from said bridging molecules is formed on the surface of said photocatalyst.

4. The material according to claim 1, further having an optically transparent substrate, wherein said photocatalyst is formed in the form of a film of thickness 1 to 7 µm on a surface of said substrate.

5. The material according to claim 4, wherein said photocatalyst layer formed in the form of a film has a transmittance to ultraviolet radiation of wavelength 250 to 400 nm of not more than 1%.

6. A method of manufacturing a photocatalytic material used in selectively inactivating a specific biologically harmful substance that may be contained in a liquid or gas to be treated, the method comprising the steps of:
preparing a photocatalyst that is able to inactivate the harmful substance through photocatalysis;
disposing bridging molecules on a surface of said photocatalyst in the form of a monolayer; and
bonding a holding substance having holding specificity of selectively holding the specific harmful substance to said bridging molecules.

7. The method according to claim 6, wherein an optically transparent substrate is prepared, and said photocatalyst layer is formed to a thickness of 1 to 7 µm on a surface of said substrate.

8. The method according to claim 7, wherein said photocatalyst layer is formed using a CVD method.

9. The method according to claim 8, wherein said photocatalyst layer is formed so as to have a transmittance to ultraviolet radiation of wavelength 250 to 400 nm of not more than 1%.

10. The method according to any one of claims 6 through 9, wherein in said step of disposing bridging molecules on a surface of said photocatalyst in the form of a monolayer, processing is carried out in which said photocatalyst is exposed to a vapor containing a coupling agent, thus bonding said coupling agent to the surface of said photocatalyst.

11. The method according to claim 10, wherein a silane coupling agent having alkoxy groups is used as said coupling agent.

12. A method of selectively inactivating a specific biologically harmful substance contained in a liquid or gas to be treated, the method comprising the steps of:
preparing the photocatalytic material according to claim 1;
making the liquid or gas to be treated come into contact with at least parts of said material containing said holding substance; and
irradiating light capable of bringing about a photocatalytic reaction onto at least parts of said material containing said photocatalyst.

13. An apparatus that treats, by photocatalysis, a specific biologically harmful substance contained in a liquid or gas to be treated, the apparatus comprising:
the photocatalytic material according to claim 1;
a flow path through which the liquid or gas containing the harmful substance is fed onto said photocatalytic material; and
a light source that irradiates light capable of bringing about a photocatalytic reaction onto at least parts of said photocatalytic material containing said photocatalyst.

14. An apparatus that treats, by photocatalysis, a specific biologically harmful substance contained in a liquid or gas to be treated, the apparatus comprising:
at least one pair of optically transparent substrates disposed separated from one another;
a material comprising a holding substance having holding specificity of selectively holding a specific biologically harmful substance, and a photocatalyst that is able to inactivate the harmful substance held by said holding substance through photocatalysis, said material being disposed on mutually facing surfaces of said substrates forming each pair;
a wall member disposed between said substrates forming each pair, said wall member provided in a state such that a fluid can flow from a portion of the space formed between said substrates on one side of said wall member to the portion of this space on the other side of said wall member;
at least one inflow port through which the liquid or gas to be treated is introduced into the space formed between said substrates forming each pair, said inflow port formed between one of said substrates and said wall member;
at least one outflow port through which the liquid or gas is discharged from the space formed between said substrates forming each pair to the outside, said outflow port formed between the other one of said substrates and said wall member; and
a light source that irradiates light capable of bringing about a photocatalytic reaction through one of said substrates onto at least parts of said material containing said photocatalyst.

15. The apparatus according to claim 14, wherein said wall member is formed so as to substantially not transmit light.

16. The apparatus according to claim 14, wherein a plurality of said inflow ports are provided at one end on one side of the space between said substrates that has been divided into two by said wall member, and a plurality of said outflow ports are provided in one end on the other side of the space between said substrates that has been divided into two.

17. An apparatus for treating, by photocatalysis, a specific biologically harmful substance contained in a liquid or gas, the apparatus comprising:
a vessel having an optically reflective inner surface;
at least one optically transparent substrate disposed inside said vessel, said substrate having formed therein a flow path through which a liquid or gas to be treated can flow;
a material that is disposed on the inside of said substrate, said material comprising a holding substance having holding specificity of being able to selectively hold a specific biologically harmful substance, and a photocatalyst that is able to inactivate the harmful substance held by said holding substance through photocatalysis; and
a light source that irradiates light capable of bringing about a photocatalytic reaction through said substrate onto at least parts of said material containing said photocatalyst.

18. The apparatus according to claim 17, wherein said light source is disposed inside said vessel, and a plurality of said substrates are provided close to said light source.

19. The apparatus according to claim 18, wherein the plurality of said substrates are connected together in series in a state such that the liquid or gas to be treated can pass therethrough.

20. The apparatus according to claim 14 or 17, wherein said holding substance contained in said material is linked to the surface of said photocatalyst via bridging molecules.

21. The apparatus according to claim 13, 14 or 17, further comprising cooling means for suppressing heat discharge from said light source.

22. The apparatus according to claim 21, having a blower that blows a gas for cooling said light source as said cooling means.
